(19) Europäisches Patentamt
European Patent Office
Office européen des brevets

(11) EP 3 990 434 B1

(12) EUROPEAN PATENT SPECIFICATION

(45) Date of publication and mention of the grant of the patent:
**25.06.2025 Bulletin 2025/26**

(21) Application number: **20830914.6**

(22) Date of filing: **25.06.2020**

(51) International Patent Classification (IPC):
***C07D 233/06*** *(2006.01)* ***C07D 401/06*** *(2006.01)*
***C07D 401/14*** *(2006.01)* ***A61K 31/4164*** *(2006.01)*
***A61K 31/4178*** *(2006.01)* ***A61K 45/06*** *(2006.01)*
***A61P 33/06*** *(2006.01)*

(52) Cooperative Patent Classification (CPC):
**C07D 401/06; A61K 45/06; A61P 33/06; C07D 233/06; C07D 401/14;** Y02A 50/30

(86) International application number:
**PCT/SG2020/050368**

(87) International publication number:
**WO 2020/263191 (30.12.2020 Gazette 2020/53)**

# (54) 1,1'-[1,3-(5-METHOXY)PHENYLENEBIS(METHYLENE)]BIS[4,5-DIPHENYL-1H-IMIDAZOLE] AND SIMILAR COMPOUNDS FOR THE TREATMENT OF MALARIA

1,1'-[1,3-(5-METHOXY)PHENYLENEBIS(METHYLENE)]BIS[4,5-DIPHENYL-1H-IMIDAZOLE] UND ÄHNLICHE VERBINDUNGEN ZUR BEHANDLUNG VON MALARIA

1,1'-[1,3-(5-MÉTHOXY)PHÉNYLÈNEBIS(MÉTHYLÈNE)]BIS[4,5-DIPHÉNYL-1H-IMIDAZOLE] ET COMPOSÉS SIMILAIRES POUR LE TRAITEMENT DU PALUDISME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.06.2019 SG 10201905970U**

(43) Date of publication of application:
**04.05.2022 Bulletin 2022/18**

(73) Proprietor: **Nanyang Technological University Singapore 639798 (SG)**

(72) Inventors:
- **TAY, Wen Jun Donald Singapore 639798 (SG)**
- **PREISER, Peter Rainer Singapore 639798 (SG)**
- **TAN, Choon Hong Singapore 639798 (SG)**

(74) Representative: **Viering, Jentschura & Partner mbB**
**Patent- und Rechtsanwälte**
**Hamborner Straße 53**
**40472 Düsseldorf (DE)**

(56) References cited:
**WO-A1-2009/048586** **WO-A1-2009/078992**
**WO-A1-2014/201161** **WO-A1-2017/069601**
**WO-A2-03/101954**

- **ÜSTÜN ELVAN ET AL: "A theoretical insight for solvent effect on myoglobin assay of W(CO) 4L2type novel complexes with DFT/TDDFT", JOURNAL OF MOLECULAR STRUCTURE, ELSEVIER AMSTERDAM, NL, vol. 1123, 6 July 2016 (2016-07-06), pages 433 - 440, XP029670118, ISSN: 0022-2860, DOI: 10.1016/ J.MOLSTRUC.2016.07.002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **LU TING ET AL: "Design and synthesis of novel chiral dihydroimidazolium cyclophanes as N-heterocyclic carbene precatalysts", TETRAHEDRON LETTERS, vol. 53, no. 48, November 2012 (2012-11-01), Amsterdam , NL, pages 6602 - 6605, XP093053203, ISSN: 0040-4039, DOI: 10.1016/j.tetlet.2012.09.116**
- **SOBIA TABASSUM ET AL: "Imidazolinium sulfonate and sulfamate zwitterions as chiral solvating agents for enantiomeric excess calculations", TETRAHEDRON ASYMMETRY, PERGAMON PRESS LTD, OXFORD, GB, vol. 22, no. 16, 29 September 2011 (2011-09-29), pages 1632 - 1639, XP028110589, ISSN: 0957-4166, [retrieved on 20111006], DOI: 10.1016/ J.TETASY.2011.09.018**
- **CHUANG-YI LIAO ET AL: "Robust and Electron-Rich cis-Palladium(II) Complexes with Phosphine and Carbene Ligands as Catalytic Precursors in Suzuki Coupling Reactions", CHEMISTRY - A EUROPEAN JOURNAL, JOHN WILEY & SONS, INC, DE, vol. 15, no. 2, 26 November 2008 (2008-11-26), pages 405 - 417, XP071829147, ISSN: 0947-6539, DOI: 10.1002/ CHEM.200801296**
- **JONES R C F ET AL: "Pyrrolo[1,2,3-de] quinoxalines: unexpected products from 1,3-dipolar cycloaddition of dihydroimidazolium ylides", TETRAHEDRON LETTERS, ELSEVIER, AMSTERDAM , NL, vol. 42, no. 23, 4 June 2001 (2001-06-04), pages 3951 - 3954, XP004249129, ISSN: 0040-4039, DOI: 10.1016/S0040-4039(01) 00617-7**
- **GOEBEL, T. ET AL.: "Search of Novel Agents for Therapy of Tropical Diseases and Human Immunodeficiency Virus", J. MED. CHEM., vol. 51, no. 2, 27 December 2007 (2007-12-27), pages 238 - 250, XP002623554, [retrieved on 20200807], DOI: 10.1021/JM070763Y**
- **WORTH, D. F. ET AL.: "Folate antagonists. 10. Synthesis and antimalarial effects of 6-[[(aryl and aralkyl)amino]methyl]-2,4- pteridinediamines and -pteridinediamine 8-oxides", J. MED. CHEM., vol. 21, no. 4, 1 April 1978 (1978-04-01), pages 331 - 337, XP055778911, [retrieved on 20200807], DOI: 10.1021/JM00202A003**
- **HE, W. ET AL.: "Halogen-Bonding-Induced Hydrogen Transfer to C=N Bond with Hantzsch Ester", ORG. LETT., vol. 16, no. 12, 6 June 2014 (2014-06-06), pages 3244 - 3247, XP055778914, [retrieved on 20200807], DOI: 10.1021/ OL501259Q**
- **DATABASE REGISTRY STN; 30 June 2014 (2014-06-30), ANONYMOUS: "1H-Imidazole, 1,1’-[1,3-phenylenebis(methylene)]bis[4,5-dihydro-4,5- diphenyl-, (4S,4’S,5S,5’S)- (CA INDEX NAME)", XP055778954, Database accession no. 1613133-63-3**
- **DATABASE PubChem PUBCHEM; 26 October 2006 (2006-10-26), ANONYMOUS: "4S )-1-Benzyl-4-phenyl-4,5-dihydro-1 H-imidazole", XP055778960, Database accession no. 11075424**


Remarks:

The complete document including Reference Table(s) and the Sequence Listing(s) can be downloaded from the EPO website

## Description

### Field of the Invention

The present disclosure provides a class of compounds and pharmaceutical compositions comprising such compounds.

References in the present description to methods of medical treatment or medical uses have to be understood as references to the compounds of the present invention for use in methods of medical treatment. Methods of medical treatment or medical uses are not encompassed by the claimed invention.

### Background of the Invention

Malaria is an infectious disease afflicting hundreds of millions people annually and causing 1 to 3 million death every year - mostly children under the age of 5. The vector-borne disease is caused by the protozoan parasite *Plasmodium,* of which the species *falciparum, vivax, ovale, malariae,* and most recently *knowlesi,* are found to infect humans. The disease is spread through a bite of the female *Anopheles* mosquito. The parasite ultimately infects and replicates within red blood cells and it is the infection and destruction of the red blood cells which causes the clinical symptoms like fever, fatigue, vomiting, seizures, coma and death associated with this disease. Currently there is no efficient malaria vaccine available meaning that drugs are the only way to cure any infection and also serve as an efficient chemical prophylaxis. Throughout the last 60 years, numerous efforts have been undertaken to eradicate malaria at a global scale. In the 1950's the WHO armed with a highly efficient antimalarial drug chloroquine and the insecticide DDT made the first attempt to eliminate the parasite. While successful in some regions the development of chloroquine resistant parasites had catastrophic consequences with millions of people killed during the resurgence of the parasite. Since then there has been a continuous race to develop new drugs against the disease. Since the introduction of antimalarials, development of drug resistant parasites has become more and more problematic. To reduce the risk of this happening more recently the use of combinatorial therapies, where two drugs are administered simultaneously was stipulated in the WHO guidelines.

WO 2017/069601 A1 (Latvian Institute of Organic Synthesis) appears to disclose substituted aminoalkylazoles as malarial aspartic protease inhibitors and a method of treating malaria using a pharmaceutical composition or a combination comprising the said compounds. In one example, WO 2017/069601 A1 discloses the following compound:

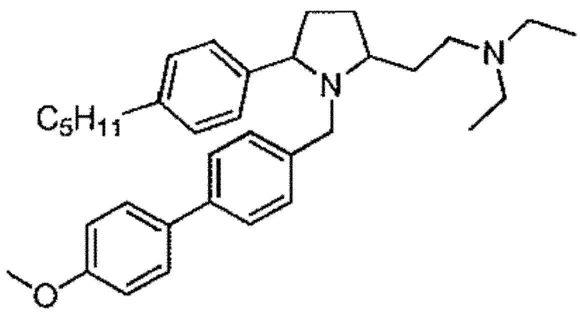

Goebel et al (J. Med. Chem., Vol. 51, No. 2, pages 238-250) appears to disclose various piperidine compounds as agents for therapy of tropical diseases and human immunodeficiency virus. In one example, Goebel et al discloses the following compound type:

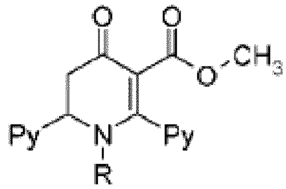

**25 - 26**

Worth et al (J. Med. Chem., Vol. 21, No. 4, pages 331-337) appears to disclose various compounds in relation with the synthesis and antimalarial effects of 6-[[(aryl and aralkyl)amino]methyl]-2,4-pteridinediamines and -pteridinediamine 8-oxides. In one example, Worth et al discloses the following compound type:

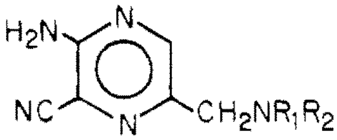

He et al (Org. Lett., Vol. 16, No. 12, pages 3244-3247) appears to disclose various compounds in relation with Halogen-Bonding-Induced Hydrogen Transfer to C=N Bond with Hantzsch Ester. In one example, He et al discloses 1,1'-[1,3-phenylenebis(methylene)]-bis[(4S,4'S,5S,5'S)-4,5-dihydro-4,5-diphenyl]-2,2'-iodo-3,3'-methyl-1H-imidazolium-bis(trifluoromethane sulfonate), 1,1'-[1,3-phenylenebis(methylene)]-bis[(4S,4'S,58,5'S)-4,5-dihydro-4,5-diphenyl]-3,3'-methyl-1H-imidazolium-bis(trifluoromethane sulfonate), 1,1'-[1,3-phenylenebis(methylene)]-bis [(4S,4'S,58,5'S)-4,5-dihydro-4,5-diphenyl]-2,2'-iodo-1H-imidazole, 1-benzyl-[(4S,5S)-4,5-dihydro-4,5-diphenyl-]-1H-imidazole and its salt form.

WO 2009/048586 A1 (Michigan State University) appears to disclose imidazoline compounds, as well as a pharmaceutical composition comprising the said compounds and its medical uses. In one example, the imidazoline compound is illustrated as follows:

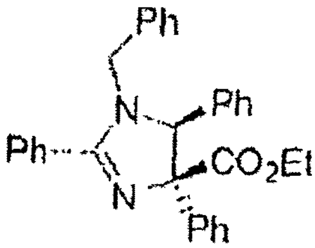

.

WO 2003/101954 A2 (Michigan State University) appears to disclose imidazoline compounds and a pharmaceutical composition thereof, as well as the medical uses of these compounds in treating inflammation. In one example, the imidazoline compound is illustrated as follows:

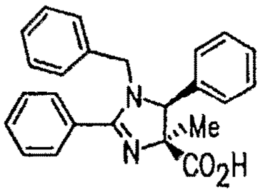

.

WO 2014/201161 A1 (Viamet Pharmaceuticals Inc [US]) appears to disclose compounds of formula (XVI), or salt thereof, as follows:

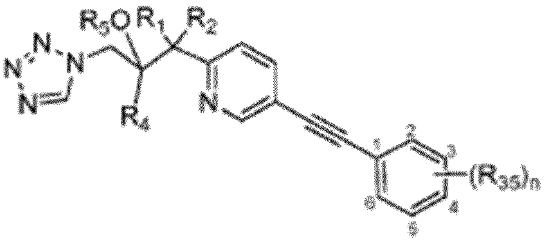

.

WO 2009/078992 A1 (Amgen Inc [US]) appears to disclose compounds of the following type:

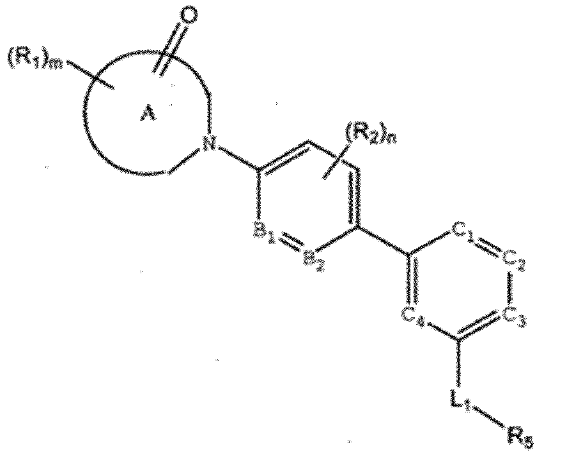

Üstün et al (Journal of Molecular Structure, vol. 1123, pages 433-440) appears to be concerned with solvent effects on a myoglobin assay of $W(CO)_4L_2$ type novel complexes with DFT/TDDFT. It appears to disclose compounds of the general type:

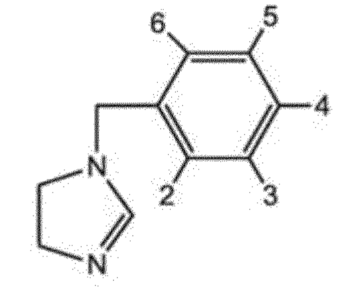

Lu et al (Tetrahedron Letters, vol. 53, no. 48, pages 6602-6605) appears to be concerned with the design and synthesis of chiral dihydroimidazolium cyclophanes as N-heterocyclic carbene precatalysts, as exemplified with the following compound:

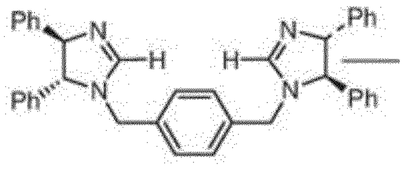

Sobia et al (Tetrahedron Asymmetry, vol. 22, no. 16, pages 1632-1639) appears to be concerned with imidazolinium sulfonate and sulfamate zwitterions as chiral solvating agents for enantiomeric excess calculations, as exemplified with the following compound:

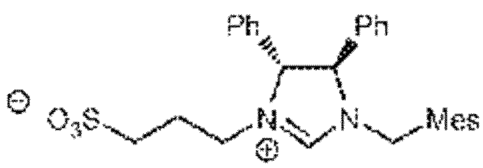

12

Chuang et al (Chemistry-A European Journal, vol. 15, no. 2, pages 405-417) appears to be concerned with cis-Palladium(II) complexes with phosphine and carbene ligands as catalytic precursors in Suzuki Coupling Reactions. An example of the carbene ligand (precursor) is illustrated as follows:

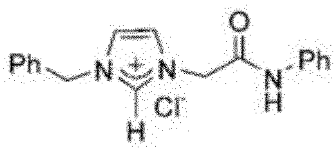

1

.

Jones et al (Tetrahedron Letters, vol. 42, no. 23, pages 3951-3954) appears to be concerned with pyrrolo[1,2,3-de]quinoxalines obtained from 1,3-dipolar cycloadditions of dihydroimidazolium ylides. An example of the dihydroimidazolium ylides is illustrated as follows:

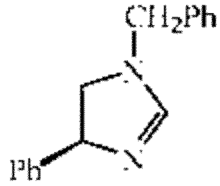

.

The discovery of artemisinin, and its other derivatives such as artesunate and dihydroartemisinin as highly effective antimalarials was a major breakthrough in the fight against the disease. While initially administered as artemisinin monotherapy this was soon abandoned and artemisinin combination therapies (ACT), such as Artemether and Lumefantrine (marketed as Coartem by Novartis), were introduced into the market with very good results.

The introduction of artemisinin as a highly effective antimalarial in combination with a suitable partner drug for the first time provided health workers with a very efficient tool to fight the disease and ACT appears to be a main reason for the significant reduction in malaria deaths globally.

Unfortunately, recently ACT has now shown signs of failing in SE Asia with resistance to artemisinin and different partner drugs becoming more and more common. Recent reports of resistant parasites from Africa indicate that drug resistance is now also found beyond SE Asia.

While there are a number of new drugs in different stages of clinical development, with ganaplacide (KAF156) from Novartis having entered phase IIB clinical trials, recrudescence of infection as well as quick development of resistance make malaria treatment still challenging and there is still need for the constant development of novel compounds that have antimalarial activity.

**Summary**

In one aspect, the present disclosure provides a compound of Formula (I)

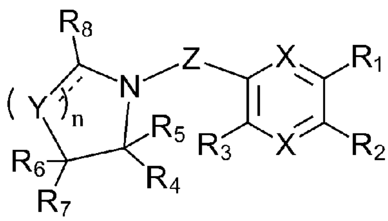

(I)

or a pharmaceutically acceptable salt thereof,
wherein

$R_1$ is selected from the group consisting of $R_2$ and

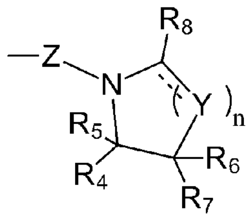

each $R_2$, $R_3$, $R_4$, $R_7$ and $R_8$ are independently selected from H, optionally substituted $C_{1-10}$ alkyl, optionally substituted $C_{2-10}$ alkenyl, optionally substituted $C_{2-10}$ alkynyl, optionally substituted $C_{3-8}$ cycloalkyl, optionally substituted 5-10 membered heteroalicyclic ring, optionally substituted $C_{6-14}$ aryl, optionally substituted 5-14 membered heteroaryl, halogen, cyano, nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$, $-C(=O)R_9$,

$R_5$ and $R_6$ are independently selected from optionally substituted benzyl or optionally substituted phenyl;

wherein one X is N and the other is CH, or one X is $COR_9$ and the other is CH;

each Y is independently selected from $C-R_b$, $C-(R_b)_2$, $N-R_b$ and N;

each Z is independently selected from bivalent $C_{1-4}$ alkyl groups, preferably $-CH_2-$, and $-(CH_2)_2-$; $R_a$ and $R_b$ are independently selected from H, optionally substituted $C_{1-10}$ alkyl, optionally substituted $C_{2-10}$ alkenyl, optionally substituted $C_{2-10}$ alkynyl, optionally substituted $C_{3-8}$ cycloalkyl, optionally substituted 5-10 membered heteroalicyclic ring, optionally substituted $C_{6-14}$ aryl, optionally substituted 5-14 membered heteroaryl, halogen, cyano, nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$, and $-C(=O)R_9$;

$R_9$ and $R_{10}$ are independently selected from H and $C_{1-10}$ alkyl, $C_{2-10}$ alkenyl, $C_{2-10}$ alkynyl, $C_{3-8}$ cycloalkyl, 5-10 membered heteroalicyclic ring, $C_{6-14}$ aryl, 5-14 membered heteroaryl, and combinations thereof;

n is 1 or 2; and

" - - -" indicates a single or double bond.

In various examples, the compound 1-({*m*-[(4,5-Diphenyl-1-imidazolinyl)methyl]phenyl}methyl)-4,5-diphenylimidazoline, i.e.

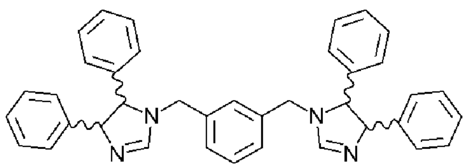

is excluded from the claimed compounds and serves as a reference compound.

In various embodiments of these compounds one X is N and the other is CH. In still further embodiments, one X is CH and the other is $CR_a$, with $R_a$ being $-OR_9$.

In various embodiments, Z is $-CH_2-$.

In the compound of the disclosure, in various embodiments, n is 1, Y is N, and "- - -" is a double bond. Alternatively, n may be 1, Y may be $CR_b$, preferably CH, and "- - -" may be a single bond. In various embodiments, if n is 2, both Y are $C-(R_b)_2$ and " - - - " is a single bond.

In various embodiments, $R_1$ is

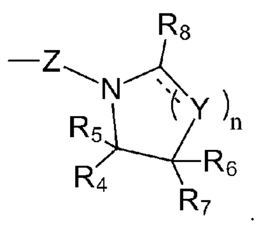

In such embodiments, Z and $R_4$-$R_8$ are defined as above. In specific embodiments thereof, the compound is symmetrical in that both Z, both $(Y)_n$, both $R_4$, both $R_5$, both $R_6$ and both $R_7$ are identical.

In various embodiments, at least one of $R_5$ and $R_6$, preferably both, are unsubstituted or substituted phenyl, preferably unsubstituted phenyl, and $R_4$ and $R_7$ are both hydrogen.

In various embodiments, $R_8$ is H.

In various embodiments, the compound is selected from any one of the following compounds:

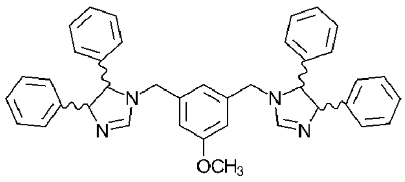

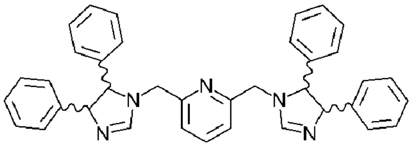

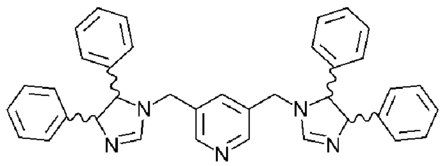

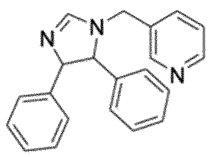

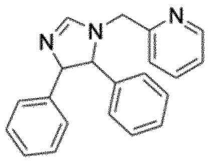

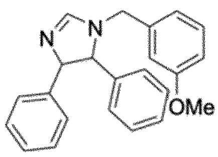

.

In another aspect, the disclosure relates to the compounds disclosed herein for use as a medicament or pharmaceutical.

In a further aspect, the disclosure is directed to a pharmaceutical composition comprising one or more compound(s) of the disclosure and a pharmaceutically acceptable excipient or carrier. The pharmaceutical composition may further comprise at least one other anti-malarial drug, for example selected from artemisinin, artesunate, dihydroartemisin, artemotil, lumefantrine, artemether, chloroquine, hydroxychloroquine, amodiaquine, mefloquine, sulfadoxine/pyrimethamine, piperaquine, primaquine, tafenoquine, and ganaplacide.

In still another aspect, the disclosure is directed to one or more compounds of the disclosure or the pharmaceutical composition of the disclosure for use in a method for preventing or treating malaria in a subject in need thereof. This aspect also covers uses of the compounds or pharmaceutical compositions of the disclosure for the manufacture of a medicament for the treatment or prevention of malaria in a subject in need thereof, wherein said prevention or treatment may comprise administering a therapeutically or prophylactically effective amount of the compounds or pharmaceutical compositions of the disclosure.

**Brief description of the drawings**

**Figure 1: Regression analysis of the dose response curve of compound A1.** Efficacy values displayed in the table. IC50, IC90, and IC99 values are in μM. Analysis was done using ICEstimator 1.2.

**Figure 2: Stage activity essay of A1.** Top row of panels show smears of parasite growth over a course of one life cycle without treatment. Subsequent rows show treatment with compound A1 given at 0, 12, 24, and 36 hours of the life cycle, with the effect on parasite growth being monitored every 12 hours.

**Figure 3: Anti-gametocyte activity of A1.** Left panel shows the condensed or lysed morphologies of the gametocytes post-A1 treatment compared to the healthy morphology. These morphologies were used to determine viability of the gametocytes in the cell count. Right panel shows the proportion of live or dead gametocytes in each treatment.

**Figure 4: Protein hits from ITDR CETSA screen for A1 targets.** Four were ribosomal proteins and another a zinc-finger protein (PF3D7_1315400).

**Figure 5: CETSA validation of the putative zinc finger target protein. A**) Fluorescent Western Blot of 100μM treated vs non-treated lysates. Lanes from left to right are lysates heat-treated with increasing temperatures. 680 (red) and 800nm (green) bands are aldolase and zinc finger proteins respectively. **B, C**) Relative band intensity of Zinc Finger (CCCH-type) protein normalized against the aldolase protein.

**Figure 6: *In vivo* efficacy of A1 against the rodent malaria parasite, *P. berghei,* in a BALB/c model.** When treated at 30mg/kg once daily for four days, compound A1 was able to reduce parasitemia to below the level of detection. In comparison, the decrease of parasitemia by artemisinin was slower, and was unable to further reduce parasitemia beyond day 3. The same was observed for A1 when mice were treated at 20mg/kg. 5 and 10mg/kg had very weak or no effect at all. Parasitemia was measured by flow cytometry using a transgenic GFP-expressing cell line.

**Figure 7: Evaluation of analogues. A)** Commercially available reference compounds and **B)** newly synthesized analogues of the reference compound A1 and their *in vitro* anti-parasitic activity.

**Figure 8: *In vivo* 4-day suppressive test of compound A1 and its analogues.** SAR 13 showed the highest activity at 20mg/kg (center column, grey bar), while compound A1 required a 30mg/kg dose in order to achieve the same level of efficacy (left column, yellow bar). SAR 14 however, was not able to achieve full inhibition even at 30mg/kg (right column). SAR14: 1-({6-[(4,5-Diphenyl-1-imidazolinyl)methyl]-2-pyridyl}methyl)-4,5-diphenylimidazoline; SAR13: 1-({3-[(4,5-Diphenyl-1-imidazolinyl)methyl]-5-methoxyphenyl}methyl)-4,5-diphenylimidazoline.

**Figure 9: Compound A1 inhibits the proteolytic activity of FLN.** Fluorescence emission of cleaved peptide substrates were measured at 490nm over 7min. FLN activity was abolished with 10μM A1 (green), similar to that of the positive control 1mM ZB1 (dark blue). No inhibition of proteolytic activity was observed in cyclohexamine (CHM, red), similar to the solvent controls (DMSO, purple; methanol, light blue; water, orange).

## Detailed description

Embodiments of the present disclosure are described below.

Definitions

Unless otherwise stated, the following terms used in the specification and claims have the meanings disclosed below:
"At least one", as used herein in relation to any component, refers to the number of chemically different molecules, i.e. to the number of different types of the referenced species, but not to the total number of molecules.

"One or more", as used herein, relates to at least one and comprises 1, 2, 3, 4, 5, 6, 7, 8, 9 or more of the referenced species. Similarly, "at least one" means one or more, i.e. 1, 2, 3, 4, 5, 6, 7, 8, 9 or more.

In the present specification, the terms "a" and "an" and "at least one" are the same as the term "one or more" and can be employed interchangeably.

"About", as used herein in relation to a numerical value, means said value ±10%, preferably ±5%.

All percentages given herein in relation to the compositions or formulations relate to weight % relative to the total weight of the respective composition or formula, if not explicitly stated otherwise.

"Alkyl" refers to a saturated aliphatic hydrocarbon including straight chain, or branched chain groups. Preferably, the alkyl group has 1 to 10 carbon atoms (whenever a numerical range; e.g.,"1-10", is stated herein, it means that the group, in this case the alkyl group, may contain 1 carbon atom, 2 carbon atoms, 3 carbon atoms, etc. up to and including 10 carbon atoms). More specifically, it may be a medium size alkyl having 1 to 6 carbon atoms or a lower alkyl having 1 to 4 carbon atoms e. g., methyl, ethyl, n-propyl, isopropyl, butyl, iso-butyl, tert-butyl and the like. The alkyl group may be substituted or unsubstituted. When substituted, the substituent group(s) is one or more, for example one or two groups, individually selected from the group consisting of $C_2$-$C_{10}$ alkenyl, $C_2$-$C_{10}$ alkynyl, $C_3$-$C_8$ cycloalkyl, $C_6$-$C_{14}$ aryl, 5-14 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, $C_1$-$C_{10}$ alkoxy, $C_3$-$C_8$ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, O-carboxy, nitro, sulfinyl, sulfonyl, amino, and $-NR^{11}R^{12}$ where $R^{11}$ and $R^{12}$ are independently selected from the group consisting of hydrogen, $C_1$-$C_4$ alkyl, $C_3$-$C_8$ cycloalkyl, $C_6$-$C_{14}$ aryl, carbonyl, acetyl, sulfonyl, amino, and trifluoromethanesulfonyl, or $R^{11}$ and $R^{12}$, together with the nitrogen atom to which they are attached, combine to form a five-or six-membered heteroalicyclic ring. One example of a substituted alkyl group is, without limitation, a benzyl group. Substituted alkyl also includes heteroalkyl where at least one carbon atom of a given alkyl group is replaced by a heteroatom, such as N, O or S. In such heteroalkyl groups not all carbon atoms may be replaced by heteroatoms.

A "cycloalkyl" group refers to an all-carbon monocyclic ring (i.e., rings which share an adjacent pair of carbon atoms) of 3 to 8 ring atoms wherein one of more of the rings does not have a completely conjugated pi-electron system, but may comprise one or more double bonds, e.g., cyclopropyl, cyclobutyl, cyclopentyl, cyclohexyl, cyclobutenyl, cyclopentenyl, cyclohexenyl, and the like. Examples, without limitation, of cycloalkyl groups are cyclopropane, cyclobutane,

cyclopentane, cyclopentene, cyclohexane, adamantane, cyclohexadiene, cycloheptane and, cycloheptatriene. A cycloalkyl group may be substituted or unsubstituted. When substituted, the substituent group(s) is one or more, for example one or two groups, individually selected from $C_1$-$C_{10}$ alkyl, $C_2$-$C_{10}$ alkenyl, $C_2$-$C_{10}$ alkynyl, $C_3$-$C_8$ cycloalkyl, $C_6$-$C_{14}$ aryl, 5-14 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, $C_1$-$C_{10}$ alkoxy, $C_3$-$C_8$ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, O-carboxy, nitro, sulfinyl, sulfonyl, amino, and $-NR^{11}R^{12}$ where $R^{11}$ and $R^{12}$ are as defined above.

An "alkenyl" group refers to an alkyl group, as defined herein, consisting of at least two carbon atoms and at least one carbon-carbon double bond e. g., ethenyl, propenyl, butenyl or pentenyl and their structural isomeric forms such as 1- or 2-propenyl, 1-, 2-, or 3-butenyl and the like. If substituted, the substituents are selected as disclosed for "alkyl" above and also include heteroalkenyl.

An "alkynyl" group refers to an alkyl group, as defined herein, consisting of at least two carbon atoms and at least one carbon-carbon triple bond e. g., acetylene, ethynyl, propynyl, butynyl, or pentynyl. If substituted, the substituents are selected as disclosed for "alkyl" above.

An "aryl" group refers to an all-carbon monocyclic or fused-ring polycyclic (i.e., rings which share adjacent pairs of carbon atoms) groups of 6 to 14 ring atoms and having a completely conjugated pi-electron system. Examples, without limitation, of aryl groups are phenyl, naphthalenyl and anthracenyl. The aryl group may be substituted or unsubstituted. When substituted, the substituted group(s) is one or more, for example one, two, or three substituents, independently selected from the group consisting of $C_1$-$C_{10}$ alkyl, $C_2$-$C_{10}$ alkenyl, $C_2$-$C_{10}$ alkynyl, $C_3$-$C_8$ cycloalkyl, $C_6$-$C_{14}$ aryl, 5-14 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, $C_1$-$C_{10}$ alkoxy, $C_3$-$C_8$ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, sulfinyl, sulfonyl, amino, and $-NR^{11}R^{12}$ where $R^{11}$ and $R^{12}$ are as defined above. In various embodiments, the substituent(s) is/are independently selected from chloro, fluoro, bromo, methyl, ethyl, hydroxy, methoxy, nitro, carboxy, methoxycarbonyl, sulfonyl, or amino.

A "heteroaryl" group refers to a monocyclic or fused aromatic ring (i.e., rings which share an adjacent pair of atoms) of 5 to 14 ring atoms in which one, two, three or four ring atoms are selected from the group consisting of nitrogen, oxygen and sulphur, or optionally other heteroatoms, and the rest being carbon. Examples, without limitation, of heteroaryl groups are pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-triazinyl, 1,2,3-triazinyl, benzofuryl, isobenzofuryl, benzothienyl, benzotriazolyl, isobenzothienyl, indolyl, isoindolyl, 3H-indolyl, benzimidazolyl, benzothiazolyl, benzoxazolyl, quinolizinyl, quinazolinyl, pthalazinyl, quinoxalinyl, cinnnolinyl, napthyridinyl, quinolyl, isoquinolyl, tetrazolyl, 5,6,7,8-tetrahydroquinolyl, 5, 6, 7, 8-tetra-hydroisoquinolyl, purinyl, pteridinyl, pyridinyl, pyrimidinyl, carbazolyl, xanthenyl or benzoquinolyl. The heteroaryl group may be substituted or unsubstituted. When substituted, the substituted group(s) is one or more, for example one or two substituents, independently selected from the group consisting of $C_1$-$C_{10}$ alkyl, $C_2$-$C_{10}$ alkenyl, $C_{2-}C_{10}$ alkynyl, $C_3$-$C_8$ cycloalkyl, $C_6$-$C_{14}$ aryl, 5-14 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, $C_1$-$C_{10}$ alkoxy, $C_3$-$C_8$ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, sulfinyl, sulfonyl, amino, and $-NR^{11}R^{12}$ with $R^{11}$ and $R^{12}$ being as defined above. In various embodiments, the substituent(s) is/are independently selected from chloro, fluoro, bromo, methyl, ethyl, hydroxy, methoxy, nitro, carboxy, methoxycarbonyl, sulfonyl, or amino.

A "heteroalicyclic" group refers to a monocyclic or fused ring of 5 to 10 ring atoms containing one, two, or three heteroatoms in the ring which are selected from the group consisting of nitrogen, oxygen and $-S(O)_n$ where n is 0-2, or optionally other heteroatoms, the remaining ring atoms being carbon. The rings may also have one or more double bonds. However, the rings do not have a completely conjugated pi-electron system. Examples, without limitation, of heteroalicyclic groups are pyrrolidine, piperidine, piperazine, morpholine, imidazolidine, tetrahydropyridazine, tetrahydrofuran, thiomorpholine, tetrahydropyridine, and the like. The heteroalicyclic ring may be substituted or unsubstituted. When substituted, the substituted group (s) is one or more, for example one, two, or three substituents, independently selected from the group consisting of $C_1$-$C_{10}$ alkyl, $C_2$-$C_{10}$ alkenyl, $C_2$-$C_{10}$ alkynyl, $C_3$-$C_8$ cycloalkyl, $C_6$-$C_{14}$ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, $C_1$-$C_{10}$ alkoxy, $C_3$-$C_8$ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, sulfinyl, sulfonyl, amino, and $-NR^{11}R^{12}$ with $R^{11}$ and $R^{12}$ being as defined above.

In all above defined embodiments where substituted groups are defined, it may, in various embodiments, be

preferred that the substituent group is not itself substituted. For example, if alkyl is substituted with aryl, thus forming an alkylaryl group, the aryl moiety is, in various embodiments, unsubstituted, unless specified to the contrary.

A "hydroxy" group refers to an -OH group.

An "alkoxy" group refers to an -O-unsubstituted alkyl and -O-substituted alkyl group, as defined herein. Examples include and are not limited to methoxy, ethoxy, propoxy, butoxy, and the like.

A "cycloalkoxy" group refers to an -O-cycloalkyl group, as defined herein. One example is cyclopropyloxy.

An "aryloxy" group refers to both an -O-aryl and an -O-heteroaryl group, as defined herein. Examples include and are not limited to phenoxy, napthyloxy, pyridyloxy, furanyloxy, and the like.

A "mercapto" group refers to an -SH group.

An "alkylthio" group refers to both an S-alkyl and an -S-cycloalkyl group, as defined herein. Examples include and are not limited to methylthio, ethylthio, and the like.

An "arylthio" group refers to both an -S-aryl and an -S-heteroaryl group, as defined herein. Examples include and are not limited to phenylthio, napthylthio, pyridylthio, furanylthio, and the like.

A "sulfinyl" group refers to a -S(O)-R" group, wherein, R" is selected from the group consisting of hydrogen, hydroxy, $C_1$-$C_{10}$ alkyl, $C_2$-$C_{10}$ alkenyl, $C_2$-$C_{10}$ alkynyl, $C_3$-$C_8$ cycloalkyl, $C_6$-$C_{14}$ aryl, 5-14 membered heteroaryl (bonded through a ring carbon) and 5-10 membered heteroalicyclic (bonded through a ring carbon), as defined herein.

A "sulfonyl" group refers to a $-S(O)_2R''$ group wherein, R" is as defined above.

A "trihalomethyl" group refers to a $-CX_3$ group wherein X is a halo group as defined herein e. g., trifluoromethyl, trichloromethyl, tribromomethyl, dichlorofluoromethyl, and the like.

"Carbonyl" refers to a -C(=O)-R" group, where R" is as defined above. Representative examples include and the not limited to acetyl, propionyl, benzoyl, formyl, cyclopropylcarbonyl, pyridinylcarbonyl, pyrrolidin-1-yl-carbonyl, and the like.

A "thiocarbonyl" group refers to a -C(=S)-R" group, with R" as defined herein.

"C-carboxy" and "carboxy" which are used interchangeably herein refer to a -C(=O)O-R" group, with R" as defined herein, e. g. -COOH, methoxycarbonyl, ethoxycarbonyl, benzyloxycarbonyl, and the like.

An "O-carboxy" group refers to a -OC(=O)R" group, with R" as defined herein, e.g. methylcarbonyloxy, phenylcarbonyloxy, benzylcarbonyloxy, and the like.

An "acetyl" group refers to a $-C(=O)CH_3$ group.

A "carboxylic acid" group refers to a C-carboxy group in which R" is hydrogen.

A "halo" or "halogen" group refers to fluorine, chlorine, bromine or iodine.

A "cyano" group refers to a -CN group.

A "nitro" group refers to a $-NO_2$ group.

An "O-carbamyl" group refers to a $-OC(=O)NR^{11}R^{12}$ group, with $R^{11}$ and $R^{12}$ as defined herein.

An "N-carbamyl" group refers to a $R^{12}OC$ (=O) $NR^{11}$- group, with $R^{11}$ and $R^{12}$ as defined herein.

An "O-thiocarbamyl" group refers to a $-OC(=S)NR^{11}R^{12}$ group, with $R^{11}$ and $R^{12}$ as defined herein.

An "N-thiocarbamyl" group refers to a $R^{12}OC(=S)NR^{11}$- group, with $R^{11}$ and $R^{12}$ as defined herein.

An "amino" group refers to an $-NR^{11}R^{12}$ group, wherein $R^{11}$ and $R^{12}$ are independently hydrogen or unsubstituted lower alkyl, e.g, $-NH_2$, dimethylamino, diethylamino, ethylamino, methylamino, and the like.

A "C-amido" group refers to a $-C(=O)NR^{11}R^{12}$ group, with $R^{11}$ and $R^{12}$ as defined herein. For example, $R^{11}$ is hydrogen or unsubstituted $C_1$-$C_4$ alkyl and $R^{12}$ is hydrogen, $C_1$-$C_4$ alkyl optionally substituted with heteroalicyclic, hydroxy, or amino. For example, $C(=O)NR^{11}R^{12}$ may be aminocarbonyl, dimethylaminocarbonyl, diethylaminocarbonyl, diethylaminoethylaminocarbonyl, ethylaminoethylaminocarbonyl, and the like.

An "N-amido" group refers to a $R^{12}C(=O)NR^{11}$- group, with $R^{11}$ and $R^{12}$ as defined herein, e.g. acetylamino, and the like.

An "effective amount", as used herein, relates to an amount that is sufficient to provide a desired effect, including preventing, reducing the risk of being afflicted by, alleviating and abating a disease and/or its attendant symptoms. This applies to terms used herein, such as "therapeutically effective amount" (alleviating and abating a disease and/or its attendant symptoms) and "prophylactically effective amount" (preventing, reducing the risk of being afflicted by a disease and/or its attendant symptoms).

"Prevention" as used herein, as well as related terms such as "prevent" or "preventing," is meant to refer to provide a subject not yet affected by the condition with a benefit that serves to avoid, delay, forestall, minimize, or reduce the recurrence/onset of the condition to be prevented and/or its attendant symptoms. Such preventative benefits include, for example, delaying development and/or recurrence of the condition, or reducing the duration, severity, or intensity of one or more unwanted features associated with the condition if it eventually develops.

"Treatment" as used herein, as well as related terms such as "treat" or "treating," refers to eradicating, reducing, ameliorating, or reversing a condition or one or more of the unwanted symptoms associated with the condition being treated.

By "pharmaceutically acceptable" it is meant that a particular compound or component is generally regarded as

safe and nontoxic at the levels employed.

Various compounds of the disclosure possess asymmetric carbon atoms (optical or chiral centers) or double bonds; the enantiomers, racemates, diastereomers, tautomers, geometric isomers, stereoisometric forms that may be defined, in terms of absolute stereochemistry, as (R)- or (S)-or, as (D)- or (L)- for amino acids, and individual isomers are encompassed within the scope of the disclosure. The compounds of the disclosure do not include those, which are known in the art to be too unstable to synthesize and/or isolate. The disclosure is meant to include compounds in racemic and optically pure forms. Optically active (R)- and (S)- isomers may be prepared using chiral synthons or chiral reagents, or resolved using conventional techniques. When the compounds described herein contain olefinic bonds or other centers of geometric asymmetry, and unless specified otherwise or prevented by structural constraints, it is intended that the compounds include both E and Z geometric isomers.

The term "tautomer," as used herein, refers to one of two or more structural isomers which exist in equilibrium and which are readily converted from one isomeric form to another. It will be apparent to one skilled in the art that certain compounds of the disclosure may exist in tautomeric forms, all such tautomeric forms of the compounds being within the scope of the disclosure.

Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the disclosure.

Unless otherwise stated, structures depicted herein are also meant to include compounds, which differ only in the presence of one or more isotopically enriched atoms. For example, compounds having the present structures except for the replacement of a hydrogen by a deuterium or tritium, or the replacement of a carbon by $^{13}C$- or $^{14}C$ -enriched carbon are within the scope of the disclosure.

The term "prodrug", as used herein, refers to a compound, which is in a prodrug form. Prodrugs of the compounds described herein are those compounds that readily undergo chemical changes under physiological conditions to provide the compounds.

Prodrug forms of the herein disclosed compounds are designed to improve their physicochemical properties (e.g. solubility, hydrophilicity, stability) and pharmacokinetic behavior (e.g. absorption, distribution, metabolism, excretion and toxicity). Prodrugs of the herein disclosed compounds can be designed for enrichment in the target cells, tissues or organs.

Prodrug design strategies can be carrier-linked (i.e., they carry promoieties), can comprise spacers or can represent conjugates with biomacromolecules. Prodrug forms of the herein disclosed compounds can be mono-, double-, triple- (or multiple) prodrugs as well as mono-, bi-, tri- (or multi-) functional prodrugs. They can be bioactivated by physicochemical or enzymatic mechanisms.

Additionally, prodrugs can be converted to the compounds of the disclosure by chemical or biochemical methods in an *ex vivo* environment. For example, prodrugs can be slowly converted to the compounds of the disclosure when placed in a transdermal patch reservoir with a suitable enzyme or chemical reagent.

For more concrete prodrug examples, reference is made to J. Med. Chem. 2004, 47(10):2393-404 and Nat. Rev. Drug Discov. 2018, 17(8):559-587.

A "pharmaceutical composition" refers to a mixture of one or more of the compounds described herein, or physiologically/pharmaceutically acceptable salts thereof, with other chemical components, such as physiologically/-pharmaceutically acceptable carriers (including diluents and solvents) and excipients. The purpose of a pharmaceutical composition is to facilitate administration of a compound to an organism.

The compounds of Formula (I) may also act as a prodrug. A "prodrug" refers to an agent which is converted into the parent drug *in vivo*. Prodrugs are often useful because, in some situations, they may be easier to administer than the parent drug. They may, for instance, be bioavailable by oral administration whereas the parent drug is not. The prodrug may also have improved solubility in pharmaceutical compositions over the parent drug. A prodrug may be converted into the parent drug by various mechanisms, including enzymatic processes and metabolic hydrolysis.

As used herein, a "physiologically/pharmaceutically acceptable carrier" refers to a carrier or diluent that does not cause significant irritation to an organism and does not abrogate the biological activity and properties of the. administered compound.

A "pharmaceutically acceptable excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

As used herein, the term "pharmaceutically acceptable salt" refers to those salts which retain the biological effectiveness and properties of the parent compound without being toxic to the subject. Such salts include, but are not restricted to: (1) an acid addition salt which is obtained by reaction of the free base of the parent compound with inorganic acids such as hydrochloric acid, hydrobromic acid, nitric acid, phosphoric acid, sulfuric acid, and perchloric acid and the like, or with organic acids such as acetic acid, oxalic acid, (D) or (L) malic acid, maleic acid, methanesulfonic acid,

ethanesulfonic acid, p-toluenesulfonic acid, salicylic acid, tartaric acid, citric acid, succinic acid or malonic acid and the like, preferably hydrochloric acid or (L)-malic acid; or (2) salts formed when an acidic proton present in the parent compound either is replaced by a metal ion, e. g., an alkali metal ion, such as sodium or potassium, an alkaline earth ion, such as magnesium or calcium, or an aluminum ion; or coordinates with an organic base such as ethanolamine, diethanolamine, triethanolamine, tromethamine, N-methylglucamine, and the like. For more specific, non-limiting examples see, for instance, Berge et al., "Pharmaceutical Salts", Journal of Pharmaceutical Science, 1977, 66, 1-19).

Various compounds of the disclosure can exist in non-solvated forms as well as solvated forms ("solvates"), including hydrated forms. In general, the solvated forms are functionally equivalent to non-solvated forms and are encompassed within the scope of the disclosure. Various compounds of the disclosure may exist in multiple crystalline or amorphous forms. In general, all physical forms are equivalent for the uses contemplated by and are intended to be within the scope of the disclosure.

The term "malaria" as used herein generally refers to infection with a protozoan parasite of the genus *Plasmodium,* specifically any one of the species *falciparum, vivax, ovale, malariae,* and most recently *knowlesi.* Clinical symptoms include fever, fatigue, vomiting, seizures, coma and death.

"Subject", as used herein, refers to any living entity amenable to treatment with the disclosed compounds and compositions. The subjects are typically mammals, in particular a human being.

The compound 1-({m-[(4,5-Diphenyl-1-imidazolinyl)methyl]phenyl}methyl)-4,5-diphenylimidazoline, i.e.

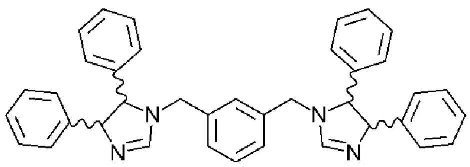

is also referred to herein as "compound A1" or "A1" and is a comparative example.

Embodiments

The present disclosure provides compounds of Formula (I)

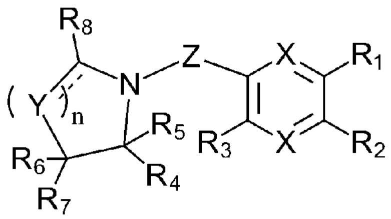

(I)

or pharmaceutically acceptable salts thereof. Also encompassed are stereoisomers, and tautomers thereof.

In these compounds, $R_1$ represents a group selected from the group consisting of $R_2$ and

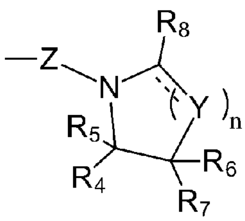

,

wherein Y, Z and $R_4$-$R_8$ are as defined below.

At each occurrence, $R_2$, $R_3$, $R_4$, $R_5$, $R_6$, $R_7$ and $R_8$ are independently selected from H, optionally substituted $C_{1-10}$ alkyl, optionally substituted $C_{2-10}$ alkenyl, optionally substituted $C_{2-10}$ alkynyl, optionally substituted $C_{3-8}$ cycloalkyl, optionally substituted 5-10 membered heteroalicyclic ring, optionally substituted $C_{6-14}$ aryl, optionally substituted 5-14 membered heteroaryl, halogen, cyano, nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$, $-C(=O)R_9$, with the proviso that at least one of $R_5$ and $R_6$ comprises a $C_{6-14}$ aryl or 5-14 membered heteroaryl group.

At each occurrence, X is independently selected from $C-R_a$ and N.

At each occurrence, Y is independently selected from $C-R_b$, $C-(R_b)_2$, $N-R_b$ and N. The selection of Y is

furthermore influenced by n and whether " - - - " is a single or double bond. If n is 1 and "- - -" is a double bond, Y is selected from C-$R_b$ and N. Alternatively, if n is 1 and Y is "- - -" is a single bond, Y is selected from C-$(R_b)_2$ and N-$R_b$. Still alternatively, if n is 2 and "- - - " is a double bond, a moiety =$Y_1$-$Y_2$- is formed, with $Y_1$ being selected from C-$R_b$ and N and $Y_2$ being selected from C-$(R_b)_2$ and N-$R_b$. Still alternatively, if n is 2 and "- - -" is a single bond, a moiety -$Y_1$-$Y_2$- is formed, with $Y_1$ and $Y_2$ being independently selected from C-$(R_b)_2$, and N-$R_b$, preferably C-$(R_b)_2$.

At each occurrence, Z is independently selected from bivalent $C_{1-4}$ alkyl groups, preferably -$CH_2$-, and -$(CH_2)_2$-. Bivalent $C_{1-4}$ alkyl groups include -$CH_2$-, and -$(CH_2)_2$- but also other linear and branched bivalent alkyl radicals.

$R_a$ and $R_b$ are independently selected from H, optionally substituted $C_{1-10}$ alkyl, optionally substituted $C_{2-10}$ alkenyl, optionally substituted $C_{2-10}$ alkynyl, optionally substituted $C_{3-8}$ cycloalkyl, optionally substituted 5-10 membered heteroalicyclic ring, optionally substituted $C_{6-14}$ aryl, optionally substituted 5-14 membered heteroaryl, halogen, cyano, nitro, -$OR_9$, -$SR_9$, -S(O)$R_9$, -S$(O)_2R_9$, -$NR_9R_{10}$, -C(=O)$NR_9R_{10}$, -$NR_9$C(=O)$R_{10}$, -OC(=O)$NR_9R_{10}$, -$NR_9$C(=O)$OR_{10}$, -$COOR_9$, and -C(=O)$R_9$, with $R_9$ and $R_{10}$ being independently selected from H and $C_{1-10}$ alkyl, $C_{2-10}$ alkenyl, $C_{2-10}$ alkynyl, $C_{3-8}$ cycloalkyl, 5-10 membered heteroalicyclic ring, $C_{6-14}$ aryl, 5-14 membered heteroaryl, and combinations thereof. "Combinations thereof", as used in this context, means that $R_9$ and $R_{10}$ may be a combination of the recited groups, such as $C_{1-10}$ alkyl substituted with $C_{6-14}$ aryl, for example benzyl.

n at each occurrence is an integer selected from 1 and 2. If n is 1 a 5-membered heteroalicyclic ring is formed and if n is 2 a 6-membered alicyclic ring is formed. Said ring may, at the position indicated by " - - - " have a double bond. If such a double bond is not present, this means that the carbon atom carrying the $R_8$ substituent additionally is bound to a hydrogen. If n is 2 and "- - -" is a single bond, the two Y-atoms may be connected by a double bond instead. In various embodiments, n is 1 and Y is C-$R_b$, C-$(R_b)_2$, N-$R_b$ or N. In various embodiments, if n is 2, both Y may preferably be C-$(R_b)_2$ or C-$R_b$, preferably C-$(R_b)_2$, i.e. in such embodiments, " - - - " is preferably a single bond.

In various embodiments of these compounds one X is N and the other is $CR_a$. In various other embodiments both X are $CR_a$. In still further embodiments, one X is CH and the other is $CR_a$. In such embodiments, where one or both X are $CR_a$, $R_a$ may be $C_{1-4}$ alkyl, halogen, haloalkyl, or -$OR_9$. In these embodiments, where $R_a$ is -$OR_9$, $R_9$ may be $C_{1-4}$ alkyl, in particular methyl or ethyl, specifically methyl. In case $R_a$ is halogen or haloalkyl, the halogen is preferably F. It may be preferred that the ring comprising the two X ring atoms does not comprise bulky substituents with the exception of the heteroalicyclic ring linked via the "Z" linker and $R^1$.

This means that $R_a$ is preferably selected from the group consisting of H, optionally substituted $C_{1-4}$ alkyl, optionally substituted $C_{2-4}$ alkenyl, optionally substituted $C_{2-4}$ alkynyl, halogen, cyano, nitro, -$OR_9$, -$SR_9$, -S(O)$R_9$, -S$(O)_2R_9$, -$NR_9R_{10}$, -C(=O)$NR_9R_{10}$, -$NR_9$C(=O)$R_{10}$, -OC(=O)$NR_9R_{10}$, -$NR_9$C(=O)$OR_{10}$, -$COOR_9$, and -C(=O)$R_9$, with $R_9$ and $R_{10}$ being independently selected from H and $C_{1-4}$ alkyl.

In various embodiments, $R_1$ is a group of formula (II)

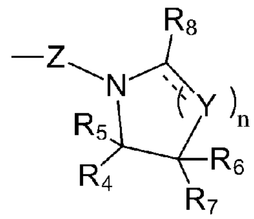

(II), with Z and $R_4$-$R_8$ being defined as above.

All embodiments disclosed herein for Z and $R_4$-$R_8$ independently apply to both the respective residues in the compound of formula (I) and in the respective residues in the group of formula (II).

In specific embodiments of $R_1$ being a group of formula (II), the compound is symmetrical in that both Z, both $(Y)_n$, both $R_4$, both $R_5$, both $R_6$ and both $R_7$ are identical. In various other embodiments, at least both Z and both $(Y)_n$ are identical.

In various embodiments, Z is -$CH_2$-.

In various embodiments, n is 1, Y is N, and " - - - " is a double bond. Alternatively, n may be 1, Y may be $CR_b$, preferably CH, and " - - - " may be a single bond.

In various embodiments, it may be preferred that n is 1 and Y is N and " - - - " is a double bond, such that the heteroalicyclic ring is a imidazoline ring.

In various embodiments, if n is 2, it may be preferred that both Y are no heteroatom, i.e. are C-$R_b$ or C-$(R_b)_2$, depending on whether a double bond is present or not. In such embodiments, the 6-membered ring is preferably fully saturated.

As regards the proviso that at least one of $R_5$ and $R_6$ comprises a $C_{6-14}$ aryl or 5-14 membered heteroaryl group, this means that at least one of $R_5$ and $R_6$ is a $C_{6-14}$ aryl or 5-14 membered heteroaryl group that may be optionally substituted or is another group substituted by a $C_{6-14}$ aryl or 5-14 membered heteroaryl group, for example alkyl, for

example, methylene, substituted by a $C_{6-14}$ aryl or 5-14 membered heteroaryl group.

As stated above, if $R_1$ is a group of formula (II), it may be preferred that both $R_5$ and/or both $R_6$ are identical.

In various embodiments, at least one of $R_5$ and $R_6$, optionally both, is/are selected from the group consisting of optionally substituted benzyl and optionally substituted phenyl. If substituted, the substituent may be selected from the group of substituents disclosed above for "aryl" groups in general. Suitable substituents include, without limitation, H, optionally substituted $C_{1-4}$ alkyl, optionally substituted $C_{2-4}$ alkenyl, optionally substituted $C_{2-4}$ alkynyl, halogen, cyano, nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$, and $-C(=O)R_9$, with $R_9$ and $R_{10}$ being independently selected from H and $C_{1-4}$ alkyl. More limited groups of suitable substituents include $C_{1-4}$ alkyl, halogen, haloalkyl, and $-OR_9$. In these embodiments, $R_9$ may be $C_{1-4}$ alkyl, in particular methyl or ethyl, specifically methyl. In case a substituent is halogen or haloalkyl, the halogen is preferably F.

In various embodiments, at least one of $R_5$ and $R_6$, preferably both, are unsubstituted or substituted phenyl, preferably unsubstituted phenyl. In these embodiments, $R_4$ and $R_7$ may be both hydrogen. Again, if $R_1$ is a group of formula (II), it may be preferred that each pair of $R_4$-$R_7$ is identical.

In various embodiments, $R_8$ is H.

In various embodiments, the compound is selected from any one of the following compounds:

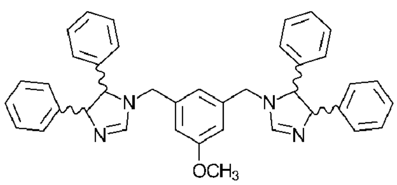

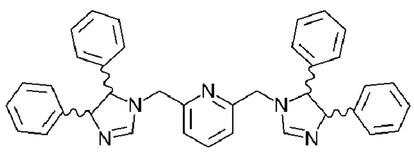

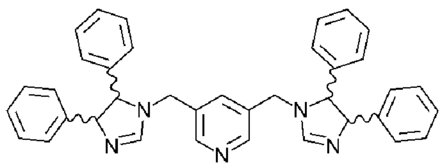

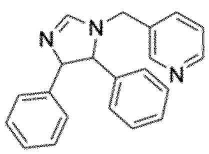

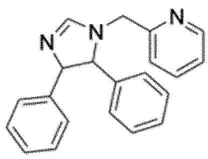

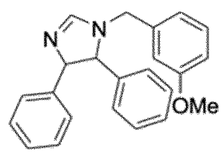

In various embodiments, the compound 1-({m-[(4,5-Diphenyl-1-imidazolinyl)methyl]phenyl}methyl)-4,5-diphenylimidazoline, i.e.

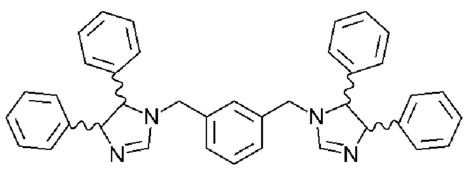

is excluded from the claimed compounds, and is considered a reference compound.

As noted above, all stereoisomers of the compounds disclosed herein are encompassed by the invention. This means that, for example, the compound

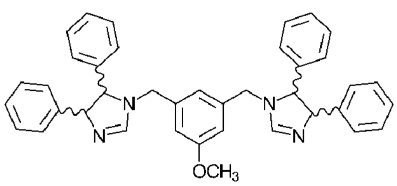

includes the stereoisomer

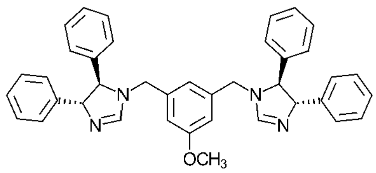

as well as all other stereoisomers that have an alternative stereochemistry, for example with respect to the pendant phenyl groups.

All uses and applications disclosed in the following with reference to the compounds of the disclosure also include the pharmaceutically acceptable salts, solvates, stereoisomers, and tautomers thereof.

In another aspect, the present disclosure relates to the use of the compounds disclosed herein as a pharmaceutical. The compounds of the disclosure are thus contemplated for use as a pharmaceutical.

In still another aspect, the disclosure is directed to one or more compounds of the disclosure for use in a method for preventing or treating malaria in a subject in need thereof. This aspect also covers uses of the compounds of the disclosure for the manufacture of a medicament for the treatment or prevention of malaria in a subject in need thereof, wherein said prevention or treatment may comprise administering a therapeutically or prophylactically effective amount of the compounds of the disclosure.

In general, compounds of the disclosure will be administered in therapeutically effective amounts via any of the usual and acceptable modes known in the art, either singly or in combination with one or more therapeutic agents. A therapeutically effective amount may vary widely depending on the severity of the disease, the age and relative health of the subject, the potency of the compound used and other factors. In general, satisfactory results are indicated to be obtained systemically at daily dosages of from about 0.03 to 2.5 mg/kg per body weight. An indicated daily dosage in a larger mammal, e.g. humans, is in the range from about 0.5 mg to about 100 mg, conveniently administered, e.g. in divided doses up to four times a day or in retard form. Suitable unit dosage forms for oral administration comprise from ca. 1 to 50 mg active ingredient.

Compounds of the disclosure can be administered as pharmaceutical compositions by any conventional route, in particular enterally, e.g., orally, e.g., in the form of tablets or capsules, or parenterally, e.g., in the form of injectable solutions or suspensions, topically, e.g., in the form of lotions, gels, ointments or creams, or in a nasal or suppository form.

The disclosure thus also relates to a pharmaceutical composition comprising one or more compound(s) of the disclosure and a pharmaceutically acceptable excipient or carrier. The carrier may include diluents and/or solvents.

Pharmaceutical compositions comprising a compound of the present disclosure in free form or in a pharmaceutically acceptable salt form in association with at least one pharmaceutically acceptable carrier or diluent can be manufactured in a conventional manner by mixing, granulating or coating methods. For example, oral compositions can be tablets or gelatin capsules comprising the active ingredient together with a) diluents, e.g., lactose, dextrose, sucrose, mannitol, sorbitol, cellulose and/or glycine; b) lubricants, e.g., silica, talcum, stearic acid, its magnesium or calcium salt and/or polyethyleneglycol; for tablets also c) binders, e.g., magnesium aluminum silicate, starch paste, gelatin, tragacanth, methylcellulose, sodium carboxymethylcellulose and or polyvinylpyrrolidone; if desired d) disintegrants, e.g., starches, agar, alginic acid or its sodium salt, or effervescent mixtures; and/or e) absorbents, colorants, flavors and sweeteners. Injectable compositions can be aqueous isotonic solutions or suspensions, and suppositories can be prepared from fatty emulsions or suspensions. The compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Suitable formulations for transdermal applications include an effective amount of a compound of the present disclosure with a carrier. A carrier can include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound to the skin of the host at a controlled and predetermined rate over a prolonged period of time, and means to secure the device to the skin. Matrix transdermal formulations may also be used. Suitable formulations for topical application, e.g., to the skin and eyes, are preferably aqueous solutions, ointments, creams or gels well-known in the art. Such may contain solubilizers, stabilizers, tonicity enhancing agents, buffers and preservatives.

Compounds of the disclosure can be administered in therapeutically effective amounts in combination with one or more therapeutic agents (pharmaceutical combinations). Non-limiting examples of compounds which can be used in combination with compounds of the disclosure are known anti-malarial drugs, for example, proguanil, chlorproguanil, trimethoprim, chloroquine, mefloquine, lumefantrine, atovaquone, pyrimethamine-sulfadoxine, pyrimethamine-dapsone, halofantrine, quinine, quinidine, amodiaquine, amopyroquine, sulphonamides, artemisinin, arteflene, artemether, artesunate, primaquine, pyronaridine, dihydroartemisin, artemotil, hydroxychloroquine, amodiaquine, piperaquine, tafenoquine, and ganaplacide.

Where the compounds of the disclosure are administered in conjunction with other therapies, dosages of the co-administered compounds will of course vary depending on the type of co-drug employed, on the specific drug employed, on the condition being treated and so forth.

The disclosure also provides for a pharmaceutical combination, e.g. a kit, comprising a) a first agent which is a compound of the disclosure as disclosed herein, in free form or in pharmaceutically acceptable salt form, and b) at least one co-agent. The kit can comprise instructions for its administration.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.

The term "pharmaceutical combination" as used herein means a product that results from the mixing or combining of more than one active ingredient and includes both fixed and non-fixed combinations of the active ingredients. The term "fixed combination" means that the active ingredients, e.g. a compound of Formula I and a co-agent, are both administered to a patient simultaneously in the form of a single entity or dosage. The term "non-fixed combination" means that the active ingredients, e.g. a compound of Formula I and a co-agent, are both administered to a patient as separate entities either simultaneously, concurrently or sequentially with no specific time limits, wherein such administration provides therapeutically effective levels of the 2 compounds in the body of the patient. The latter also applies to cocktail therapy, e.g. the administration of 3 or more active ingredients.

The pharmaceutical compositions may be used in a method for preventing or treating malaria in a subject in need thereof.

The present disclosure also includes processes for the preparation of compounds of the disclosure. In the reactions described, it can be necessary to protect reactive functional groups, for example hydroxy, amino, imino, thio or carboxy groups, where these are desired in the final product, to avoid their unwanted participation in the reactions. Conventional protecting groups can be used in accordance with standard practice, for example, see T. W. Greene and P. G. M. Wuts in "Protective Groups in Organic Chemistry", John Wiley and Sons, 1991.

Compounds of Formula I can be prepared by proceeding as generally reported in He et al. (2014) (Org. Lett. 2014, 16, 3244-3247).

All embodiments disclosed herein in relation to the compounds as such are similarly applicable to the uses and methods described herein and vice versa.

The disclosure is further illustrated by the following non-limiting examples and the appended claims.

## Examples

### Example 1: Identification of lead compound

Through a screen of a proprietary compound library of almost 680 unique compounds, the compound **A1** (4*S*,5*S*)-1-[(*m*-{[(4*S*,5*S*)-4,5-Diphenyl-1-imidazolinyl]methyl}phenyl)methyl]-4,5-diphenylimidazoline (He et al. (Org. Lett. 2014, 16, 3244-3247)) was found to be to be highly effective against wild type and multidrug-resistant malaria parasite *Plasmodium falciparum.*

For the *in vitro P. falciparum* culture, *P.falciparum* 3D7, Dd2, K1, T994, W2, and W2mef cell lines were obtained from Malaria Research and Reference Reagent Resouce Center (MR4), BEI Resources. Artemisinin-resistant and -sensitive parasite field isolates from Ratanakiri, Cambodia were obtained by request from the Tracking Resistance to Artemisinin Collaboration (TRAC). In *vitro* parasite culture was performed as described by Maier and Rug (Methods Mol Bio 2013; 923:3-15). Briefly, cultures were maintained with RPMI medium supplemented with Albumax II; 81g/L RPMI-1640 (Gibco, Life Technologies), 2.5g/L Albumax II (Gibco, Life Technologies), 2.3g/L Sodium Bicarbonate (Sigma-Aldrich), 50mg/L Hypoxanthine (Sigma-Aldrich), 10mg/L Gentamicin Sulphate (Gibco, Life Technologies), in 2-3% haematocrit human RBCs collected via patient donation at National University Hospital, Singapore. Cultures were maintained with a special mixed gas of 3% $O_2$, 5% $CO_2$, balanced with $N_2$ (SOXAL, Air Liquide, Singapore). For cultures grown in plate format, plates were kept in a sealed bag filled with mixed gas. Parasitemia and parasite stages were checked by microscopy of blood smears fixed in Methanol (Merck), stained in Giemsa (Sigma-Aldrich) in 1x Tris-Base, Acetic Acid, EDTA (TAE) buffer (analytical grade, Fischer US).

Synchronization of ring stage parasites (0-20 hour IDC) was done by incubating packed RBC culture with 5% (w/v) D-Sorbitol (Sigma-Aldrich) at 37°C for 20 minutes. Cultures were washed with incomplete RPMI (iRPMI), 2,200rpm, 3 minutes, brake 1 to remove sorbitol and cell debris. Late stage parasites (30-48 hour IDC) were isolated using a 68% Percoll (MP Biomedicals) gradient prepared with 1x Phosphate Buffer Saline (PBS) and iRPMI. Packed parasite cultures were diluted with iRPMI 1:5, then layed carefully atop of the 68% Percoll gradient in a 15mL Falcon tube. Tubes were spun at 2,200rpm for 20 minutes, brake 0. RBCs collected at the iRPMI-Percoll interface were carefully removed and washed in iRPMI twice.

In a mid-throughput compound library screen, compounds were screened for antiparasitic activity by growth rate

over a duration of 60hrs (slightly over one IDC). ~1% parasitemia of 0-3hpi ring stage culture in 2% haematocrit were treated with 10μM of library compound (0.1% final DMSO concentration), and seeded into 96well flat bottom plates. Controls of 0.1% DMSO- and 10μM Chloroquine-treated cultures, and 2% haematocrit uninfected RBCs were also done. Culture volume per well was 100μL. Plates were sealed in air-tight bags with gas mixture and incubated at 37°C. After 60hr of treatment (to allow ring stages to develop a more defined morphology), taken for Fluorescent-activated Cell Sorting (FACS) read out.

Assay read out was done by staining with Hoechst 33342 (Excitation 350nm, Emission 461nm; Molecular Probes, Life Technologies), a cell permeable DNA staining dye. Staining solution was prepared by diluting DMSO stock of Hoechst 33342 1:1000 in 1x PBS (16.2μM). Plates were spun at 2,200rpm for 5min, brake 1 to pellet cells. Culture supernatant was removed, and 100μL of staining solution was added to each well and incubated at 37°C in the dark for 20min. 200μL of cold 1x PBS was then added to the wells to dilute the dye and stop the staining. Cells were read via a High Throughput Sampler (HTS)-coupled FACS machine (BD Biosciences) in the UV laser excitation channel (Excitation 355nm).

Parasitemia obtained from test and control wells were used for the calculation of % growth inhibition of each well. % growth inhibition was calculated as 100% x (1-(Screen P% - CQ P%)/(DMSO P% -CQ P%)), where P% is parasitemia. Average % growth inhibition between replicates were calculated and plotted in Microsoft Excel. Hits displaying ≥95% growth inhibition were considered as hits.

For the dose response assay, 2-fold serial dilutions of compounds were done in 50μL starting compound treatment concentration in the 96well flat bottom plate in replicates. P. falciparum 0-3hpi ring stage parasite cultures were adjusted to 1% parasitemia in 4% haematocrit, then 50μL of culture stocks were added to the treatment wells. Controls of 0.1% DMSO- and 10μM Chloroquine-treated cultures, and 2% haematocrit uninfected RBCs were also done. Cells were incubated for 60hr before staining with Hoechst 33342 for FACS reading as per described in compound library screen. Parasitemia read outs between bioreplicates were averaged and plotted on the IC Estimator (ICE) 1.2 online software for calculation of Inhibitory Concentration 50 and 99($IC_{50}$ and $IC_{99}$) values (http://www.antimalarial-icestimator.net/MethodIn tro.htm). The results are shown in **Figure 1.**

In a second step (asexual blood stage-specificity assay), highly-synchronized parasites of 0-3hpi ring stage were either treated with 10μM of the compound or DMSO of equivalent concentrations (untreated control) at 0, 12, 24, or 36 hours of the life cycle. Thick blood smears of each treatment culture were made at 12 hour intervals. Smears were fixed with Methanol before staining with Giemsa dye for visualization on a light microscope. The tested compound showed asexual stage killing at ring and trophozoite stages. In comparison to the untreated culture, ring stage parasites formed a pyknotic structure when treated at both 0 and 12hr of the life cycle. Condensed trophozoites were seen in when treatment began at 24hr, but the compound did not seem to exert any effect when parasites at 36hr post invasion were treated, where reinvasion of cells were observed later. The results are shown in **Figure 2.** The figures show that anti-parasitic effect was observed across a large range of the life cycle of the parasite.

To assess the anti-gametocyte activity, 3D7 parasites were stress-induced into committing to the sexual stages. Culture was maintained in the presence of N-acetyl-D-Glucosamine (Sigma) for nine days to remove residual asexual parasites. Gametocytes were then treated with either the compound of the invention (A1) or lumefantrine (Sigma) over 6 days, changing the media every two days. Smears were made at the end of the treatment to determine parasitemia by cell count. The results are shown in **Figure 3.** Full gametocidal activity was observed up to 5000nM of the inventive compound, while partial inhibition was observed at 1000nM. Combined, these results provide evidence that the compound inhibits the maturation of gametocytes from stage III to V.

In a next step, the compound's *in vitro* activity was validated *in vivo* in a *P. berghei BALB/c* mice model. A growth suppression test was done by intraperitoneally infecting 4-week old *Balblc* mice with GFP-expressing parasites, and allowing the parasitemia to rise to 3-4%. Subsequently, different doses of the compound were administered intraperitoneally once daily over four days. Tail snips were done once daily to obtain blood samples for the measurement of parasitemia by FACS.

Indeed, control and reduction of parasitemia was observed in a dose dependent manner, in which the 30mpk treatment was able to fully reduce parasitemia after four days (See **Figure 6**). Recrudescence of the infection was however observed two to three days after the last dosage was given. Nevertheless, in comparison to artemisinin given at the same dosage, the A1 compound seemed to control the infection better.

In sum, it could be shown that the tested compound exhibited low nanomolar efficacy against wild-type and chloroquine-resistant *P. falciparum in vitro.* It was observed to have an anti-parasitic effect from the beginning of the parasite life cycle up to the early schizont stages. Toxicity was not observed in lung, kidney, and liver epithelial cells at 1000-folds in excess of the therapeutic dose. When tested *in vivo,* the compound was able to treat a *P. berghei* infection over multiple doses of intraperitoneal administration.

**Example 2: Target identification**

Cellular Thermal Shift Assay (CETSA® MS; https://omicscouts.com/en/cetsa-ms.html), which is based on the principle of drug-binding thermal stabilization of the protein, was employed to identify potential putative targets of the A1 compound. CETSA was performed similarly as described in Dziekan et al. 2019 (Sci Transl Med 2019; 11(473):eaau3174).

Ring (12±4hpi) and Mid-trophozoite stage synchronized (30±4hpi) *P. falciparum* 3D7 culture at ~10% parasitaemia, 2% hematocrit was used for CETSA sample preparations. To obtain stage III-V gametocytes, asexual culture of NF54 iGP2 high gametocyte-producing cell line (obtained as a gift from Till Voss, Swiss Tropical and Public Health Institute/University of Basel, Switzerland) were grown in cRPMI supplemented with 2.5μM D-glucosamine (Sigma-Aldrich) at 2-2.5% haematocrit and synchronized to a 6hr window. They were then induced for sexual stage commitment by growing in media without D-glucosamine for 1 cycle starting from ring stages, following which D-glucosamine is then replaced and media was supplemented with 10g/L N-acetyl D-glucosamine. At Day 8 post induction, Stage III gametocytes were enriched for by Percoll and washed thoroughly to remove cellular debris. Gametocytemia and cell counts were done to determine number of cells.

This step was not done for intact cell gametocytes at this stage. Parasite culture was pelleted, washed with PBS, and incubated with 10x volume of fresh 0.1% Saponin in PBS pH7.2 for 5min. Lysis reactions were centrifuged at 2500 xg for 5min, to obtain intact parasite pellet. Cells were washed three times with ice-cold 1x PBS, then resuspended in lysis buffer (50mM HEPES pH7.5, 5mM beta-glycerophosphate, 0.1mM Na3VO4, 10mM MgCl2, 2mM TCEP (only in Trophozite lysates) and cocktail EDTA-free protease inhibitors (Naclai-Tesque)). To obtain parasite lysate proteins, resuspended ring and trophozoite were lysed by three flash-freeze (liq. N2)-thawing cycles, followed by mechanical shearing with 26g and 31g needles. Samples were centrifuged at 20,000xg for 20min at 4°C to obtain soluble parasite proteins in supernatant fraction. Remaining pellet was resuspended in 1mL of lysis buffer and the procedure was repeated. The protein concentration was quantified by the BCA ProteinAssay kit (Pierce).

Ten aliquots of 100μg of proteins were added to serially diluted drug, incubated at room temperature (RT) for 3min and heated at respective temperature(s) for 3min, followed by 3min incubation at 4°C. The post-heating lysates were centrifuged at 20,000 xg for 20min at 4°C and the supernatant were collected. The protein concentration of the post-heating lysate was measured for the no drug control in ITDR or 37°C condition in melt curve experiments.

In the case of intact cell gametocyte CETSA, 4-fold serial dilutions of 10mM A1 in DMSO were done, and parasite cells were diluted to 107 cells/mL in cRPMI. Starting with the undiluted 10mM compound up to the DMSO, 3uL was carefully transferred into each well of a PCR plate. 300μL of the prepared cell stock was then added to each compound-containing well, then put into a bag and gased. Treatment was incubated for 1 hr at 37°C. After treatment, three equal 100μL wells for each treatment were made in different plates. Each plate was then sealed and subjected to 3min heat challenge, followed by 3min cooling at 4°C. Freeze-thaw and mechanical sheering as per cell lysate preparation were then done. 150μg of protein from each sample was transferred into a new tube, and added with 15μL of Hemoglobind™ resin suspension (Biotech Support Group).

Top up volume with 20mM K3PO4 (pH6.5) until 2x original volume is reached. Tubes were vortexed to mix resin, then put on constant rotation for 15min at 4°C. Tubes were centrifuge at 8,000 xg for 1min at 4°C. Protein-resin mix was resuspended and applied onto a 96well 0.22μm filter plate with an attached collection plate below. Plate was centrifuged at 800xg for 3min at 4°C. Protein concentrations of each sample were measured again using a Reducing Agent Compatible BCA Assay Kit (ThermoScientific) as per manufacturer's protocol.

After quantification, the volume equivalent to 100μg total protein in the post-heating supernatant was aliquoted and incubated with reduction and denaturation buffer containing 100mM TEAB, 20mM TCEP, 0.05% (w/v) RapiGest at 55°C for 20min, and subsequently subjected to alkylation with 55mM CAA at RT for 30 min, digestion with LysC (0.05μg of LysC/μg of protein) for 4hr and followed by trypsin digestion for 18hr at 37°C. After digestion, samples were incubated with 1% TFA for 45 min at 37°C to hydrolyze the remaining RapiGest and then spun at 20,000g for 15 min. The supernatants were collected, dried in a centrifugal vacuum evaporator and solubilized with 200mM TEAB to 1μg/μl concentration. Labeling was carried out according to the manufacturer's instruction. Briefly, 10μg of the digested protein was labeled for at least 1hr with TMT10plex Isobaric Label Reagent Set (Pierce) at a condition of pH>6 and then quenched with 1M Tris, pH7.4. The labeled samples were subsequently combined and desalted using aC18 Sep-Pak cartridge (Waters), followed by vacuum drying. Samples were resuspended in 10mM Ammonia Formate pH 10.5, 5% ACN and separated using high pH reverse phase Zorbax 300 extend C-18 4.6 mmx250mm column (Agilent) and liquid chromatography ÄKTAmicro system (GE). 96 fractions were collected and subsequently combined into 20 fractions, vacuum dried and washed again with 60% ACN, 0.1% Formic Acid followed by vacuum drying step. Collected fractions for each curve were pooled into 20 separate tubes based on the pooling scheme and dried in a centrifugal vacuum evaporator at 60°C. Each dried fraction was then washed with 100μL of 0.1% formic acid, 60% acetonitrile twice by drying. Dried fractions were then resolubilized in 10μL of 0.5% acetic acid, 0.06% TFA, 1% acetonitrile. Fractions were plated onto a 96well autosampler plate, injecting 2μL of peptide from each fraction into the LC-MS/MS.

The results are shown in **Figure 4.** Of the 5 protein hits from ITDR CETSA screen for A1 targets, four were ribosomal proteins and another a zinc-finger protein (PF3D7_1315400).

To validate the results of the screen, the Zinc Finger (CCCH) protein in 3D7 was endogenously tagged with a hemagglutinin tag and its expression validated. To do this, we amplified and cloned the 3' end region of the gene using primers F - TGACACTATAGAATACTCGCGGCCGC TGCATCTACCTTCATCAGATGCATCAAC (SEQ ID NO:1) and R - CACCAGCAGCAGCACCTCTAGCACGCGT TTCTTTGGTTTCCCATTTCCAAACTTTTG (SEQ ID NO:2). The PCR fragment was cloned into the pSLI (selection-linked integration) plasmid, obtained as a kind gift from Prof Tobias Spielmann of Bernhard Nocht Institute for Tropical Medicine, Germany. 200μL of packed infected RBCs of above 5% parasitemia (counted by blood smear) were mixed with 50μg of pSLI Pf Zinc Finger HA Tagging plasmid resuspended in 50μL 1:10 TE-EF and 250μL 2x Transfection Cytomix. Transfection mix were put into a 0.2cm cuvette (BioRad) and electroporated at 310V, 950F, ∞Ω. Electroporated cells were transferred into prewarmed flasks containing cRPMI and freshly drawn uninfected RBCs. Cuvettes were washed with media to obtain maximum number of cells. Drug selection media containing 2.5nM WR99210 (Jacobus Pharmaceutical) was changed once daily for the first five days, and subsequently once every other day until parasites were observed. Subsequently, the WR99210 selected parasites are then grown in the presence of 125μg/mL G418 (Gold Biotech) until parasites were observed.

Using 30hpi lysates from the tagged cell line, a western blot CETSA was performed and relative band intensity used to observe for thermal stabilization of the Zinc Finger protein (See **Figure 5**). These results give evidence that from a binding standpoint, the Zinc Finger (CCCH-type) protein is a putative target of the tested compound. The protein is conserved in all *Plasmodium* species.

In order to further understand how compound A1 exerts it parasitic-killing activity, it was sought to elucidate its intracellular target. Cellular Thermal Shift Assay (CETSA) was again employed to screen for potential protein targets of the compound. In this approach, the screen was conducted in stages where the compound's activity was most prominent, namely the asexual ring stage, as well as the stage III-V gametocyte stages (**Figure 9**). From both screens, multiple proteins were identified. Firstly, from the ring stages, we obtained 16 ribosomal subunit proteins, as well as 3 other non-ribosomal protein subunit hits: the putative Dipthine Synthase (PlasmoDB geneID: *PF3D7_1009000),* Falcilysin (FLN, PlasmoDB geneID: *PF3D7_1360800),* and a conserved Plasmodium protein (PlasmoDB geneID: *PF3D7_1022100*)*,* all of which passed the three median absolute deviation (3 MAD) cut-off. In gametocytes however, no ribosomal proteins were observed amongst the hits. Rather contrastingly, only two proteins were identified; FLN, as well as the Heat Shock Protein 90 (Hsp90, PlasmoDB geneID: *PF3D7_0708400*)*,* the latter only passing the 2MAD cutoff. Taken together, a single protein was identified with confidence between both stages; Falcilysin (FLN, PlasmoDB geneID: *PF3D7_1360800*).

FLN has been prominently characterized to be part of the haemoglobin digestion pathway in asexual parasites, while there have also been suggestions of an alternate role in processing proteins for apicoplast-targeting. In order to validate the results obtained from the CETSA screen, a cell-free enzymatic inhibition assay using recombinant-FLN and a fluorescent-quencher tagged 10-peptide substrate of FLN was employed. Recombinant FLN and the Dabcyl-HKRHSFRMRG (SEQ ID NO:3)-Edans fluorescent-quencher peptides were a gift from Zbynek Bozdech of School of Biological Sciences, NTU. Briefly, recombinant FLN at a final concentration of 0.3 μg/ml in assay buffer (50 mM Bis-tris, pH 7.2) was prepared. A final concentration of 5μM Dabcyl-HKRHSFRMRG (SEQ ID NO:3)-EDANS was mixed with either 10μM A1, 1mM ZB1, 1mM cyclohexamide, 0.1% DMSO, 0.1% Methanol, or water, and was added to each well of a black flat-bottom polystyrene 96-well plate (Greiner). Just prior to the read out, recombinant FLN in assay buffer was added. V0 was determined by monitoring fluorescence (λex = 340 nm, λem = 490) over 7min on a Tecan Infinite M200 Pro plate reader. Assays were done with two technical replicates each. Data of three biological replicates were averaged and plot on Microsoft excel to compare the slope of the fluorescence signal. From these assays, it could be observed that the compound A1, as well as the positive control ZB1, were able to inhibit the proteolytic activity of FLN (**Figure 9,** ZB1 data not shown). This shows that A1 acts on FLN by inhibiting its proteolytic activity.

Subsequently, in order to identify the site of FLN with which compound A1 interacts, the recombinant protein was co-crystallized with the compounds separately. In the case of each compound, A1, SAR 13, and SAR 14 (see Example 3 below), were observed to bind to FLN at the same hydrophobic pocket of FLN, although only through a very minimal contact site (data not shown).

## Example 3: Structural optimization

Four components of the parent compound were identified which have potential for chemical modification for structural activity relation (SAR) studies, namely the side heterocycles, the side phenyls, linker arms, as well as the central phenyl.

The object was to improve the antiparasitic efficacy of the compound while also improving the drug-like properties of A1. Based on predicted *in silico* pharmacokinetic properties, a key area to be improved is the solubility of the compound. Hence, a range of commercially available as well as in-house synthesized analogues were tested (**Figure 7**). While the commercially available analogues showed no significant growth inhibition at all (and are therefore considered to be

reference compounds), modification of the linker arms showed negative impact (see reference compound as top entry in Figure 7B) and modification of the central phenyl a significant impact on $IC_{50}$ values (2fold increase in activity, compounds of the invention). Interestingly, comparison with the imidazole variant (reference compound), (4*S*,5*S*)-1-[(*m*-{[(4*S*,5*S*)-4,5-Diphenyl-1-imidazolyl]methyl}phenyl)methyl]-4,5-diphenylimidazole, showed a decrease of affinity from 120 nM to 4690 nM. Similarly, replacement of the methylene linker group by a carbonyl linker group reduced affinity from 120 nM to 5042 nM, while substitution of the central phenyl ring increased affinity (**Figure 7B**).

Compound A1 (as a reference compounds) was tested against its more potent analogues 1-({6-[(4,5-Diphenyl-1-imidazolinyl)methyl]-2-pyridyl}methyl)-4,5-diphenylimidazoline (SAR14) and 1-({3-[(4,5-Diphenyl-1-imidazolinyl) methyl]-5-methoxyphenyl}methyl)-4,5-diphenylimidazoline (SAR13), in a four-day suppression tests. Four-week-old *Balb/c* mice were challenged with $10^7$ *P. berghei* parasites intravenously on Day 0. Mice were then treated with either A1, SAR 13, or SAR 14 once daily for four days from Days 1 to 4. Parasitemia was measured on Day 5, and the following formula was used to measure *in vivo* activity;

$$Activity\ (\%) = 100 - \left(\frac{Mean\ Parasitemia\ Treated}{Mean\ Parasitemia\ Untreated} \text{ X } 100\right).$$

The results are shown in **Figure 8**. It was observed that to achieve >95% killing, A1 as well as SAR 14 required a dose of 30mg/kg, while in contrast, SAR 13 only required 20mg/kg. While SAR 14 does not follow the trend of improved efficacy shown *in vitro,* it was observed that SAR 13 does.

## Claims

1. Compound of formula (I)

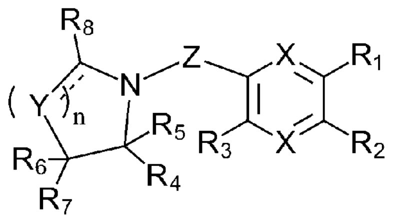

(I)

or a pharmaceutically acceptable salt thereof, wherein
$R_1$ is selected from the group consisting of $R_2$ and

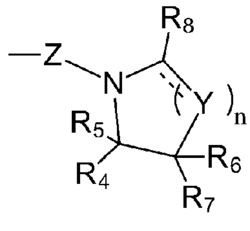

**each** $R_2$, $R_3$, $R_4$, $R_7$ and $R_8$ are independently selected from H, optionally substituted $C_{1-10}$ alkyl, optionally substituted $C_{2-10}$ alkenyl, optionally substituted $C_{2-10}$ alkynyl, optionally substituted $C_{3-8}$ cycloalkyl, optionally substituted 5-10 membered heteroalicyclic ring, optionally substituted $C_{6-14}$ aryl, optionally substituted 5-14 membered heteroaryl, halogen, cyano, nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$, $-C(=O)R_9$,
$R_5$ and $R_6$ are independently selected from optionally substituted benzyl or optionally substituted phenyl;
wherein one X is N and the other is CH, or one X is $COR_9$ and the other is CH;
each Y is independently selected from $C-R_b$, $C-(R_b)_2$, $N-R_b$ and N;
each Z is independently selected from bivalent $C_{1-4}$ alkyl groups, preferably $-CH_2-$, and $-(CH_2)_2-$; $R_a$ and $R_b$ are independently selected from H, optionally substituted $C_{1-10}$ alkyl, optionally substituted $C_{2-10}$ alkenyl, optionally substituted $C_{2-10}$ alkynyl, optionally substituted $C_{3-8}$ cycloalkyl, optionally substituted 5-10 membered hetero-alicyclic ring, optionally substituted $C_{6-14}$ aryl, optionally substituted 5-14 membered heteroaryl, halogen, cyano, nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$, and $-C(=O)R_9$;

$R_9$ and $R_{10}$ are independently selected from H and $C_{1-10}$ alkyl, $C_{2-10}$ alkenyl, $C_{2-10}$ alkynyl, $C_{3-8}$ cycloalkyl, 5-10 membered heteroalicyclic ring, $C_{6-14}$ aryl, 5-14 membered heteroaryl, and combinations thereof;
n is 1 or 2; and
" - - - " indicates a single or double bond,

wherein the substituent group(s) in substituted $C_{1-10}$ alkyl are one or more substituents, individually selected from the group consisting of $C_2$-$C_{10}$ alkenyl, $C_2$-$C_{10}$ alkynyl, $C_3$-$C_8$ cycloalkyl, $C_6$-$C_{14}$ aryl, 5-14 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, $C_1$-$C_{10}$ alkoxy, $C_3$-$C_8$ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, O-carboxy, nitro, sulfinyl, sulfonyl, amino, and $-NR^{11}R^{12}$ where $R^{11}$ and $R^{12}$ are independently selected from the group consisting of hydrogen, $C_1$-$C_4$ alkyl, $C_3$-$C_8$ cycloalkyl, $C_6$-$C_{14}$ aryl, carbonyl, acetyl, sulfonyl, amino, and trifluoromethanesulfonyl, or $R^{11}$ and $R^{12}$, together with the nitrogen atom to which they are attached, combine to form a five-or six-membered heteroalicyclic ring; substituted $C_{1-10}$ alkyl also includes heteroalkyl where at least one carbon atom of a given alkyl group is replaced by a heteroatom, such as N, O or S;
wherein the substituent group(s) in substituted $C_{2-10}$ alkenyl are as disclosed above for substituted $C_{1-10}$ alkyl with an additional substituent being heteroalkenyl;

wherein the substituent group(s) in substituted $C_{2-10}$ alkynyl are as disclosed above for substituted $C_{1-10}$ alkyl,

wherein the substituent group(s) in substituted $C_{3-8}$ cycloalkyl are as disclosed above for substituted $C_{1-10}$ alkyl with an additional substituent being $C_{1-10}$ alkyl,
wherein substituent group(s) in substituted 5-10 membered heteroalicyclic ring is one or more substituents, independently selected from the group consisting of $C_1$-$C_{10}$ alkyl, $C_2$-$C_{10}$ alkenyl, $C_2$-$C_{10}$ alkynyl, $C_3$-$C_8$ cycloalkyl, $C_6$-$C_{14}$ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, $C_1$-$C_{10}$ alkoxy, $C_3$-$C_8$ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, sulfinyl, sulfonyl, amino, and $-NR^{11}R^{12}$ with $R^{11}$ and $R^{12}$ being as defined above,
wherein substituent group(s) in substituted $C_{6-14}$ aryl are one or more substituents, independently selected from the group consisting of $C_1$-$C_{10}$ alkyl, $C_2$-$C_{10}$ alkenyl, $C_2$-$C_{10}$ alkynyl, $C_3$-$C_8$ cycloalkyl, $C_6$-$C_{14}$ aryl, 5-14 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, $C_1$-$C_{10}$ alkoxy, $C_3$-$C_8$ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, sulfinyl, sulfonyl, amino, and $-NR^{11}R^{12}$ where $R^{11}$ and $R^{12}$ are as defined above,
wherein substituent group(s) in substituted 5-14 membered heteroaryl are one or more substituents, independently selected from the group consisting of $C_1$-$C_{10}$ alkyl, $C_2$-$C_{10}$ alkenyl, $C_2$-$C_{10}$ alkynyl, $C_3$-$C_8$ cycloalkyl, $C_6$-$C_{14}$ aryl, 5-14 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, $C_1$-$C_{10}$ alkoxy, $C_3$-$C_8$ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, sulfinyl, sulfonyl, amino, and $-NR^{11}R^{12}$ with $R^{11}$ and $R^{12}$ being as defined above.

**2.** The compound of claim 1, wherein Z is $-CH_2-$.

**3.** The compound of claim 1 or 2, wherein n is 1, Y is N, and " - - - " is a double bond.

**4.** The compound of claim 1 or 2, wherein n is 1, Y is $CR_b$, preferably CH, and " - - - " is a single bond.

**5.** The compound of any one of claims 1 to 4, wherein $R_1$ is

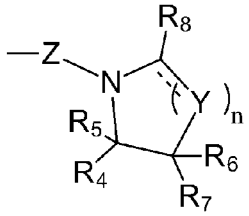

.

6. The compound of claim 5, wherein the compound is symmetrical in that both Z, both $(Y)_n$, both $R_4$, both $R_5$, both $R_6$ and both $R_7$ are identical.

7. The compound of any one of claims 1 to 6, wherein both $R_5$ and $R_6$ are unsubstituted phenyl, and $R_4$ and $R_7$ are both hydrogen.

8. The compound of any one of claims 1 to 7, wherein $R_8$ is H.

9. The compound of any one of claims 1 to 8, wherein the compound is selected from any one of the following compounds:

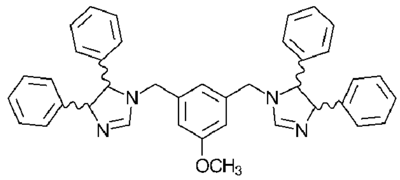

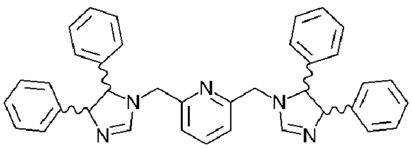

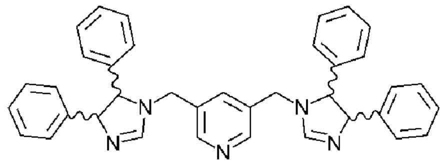

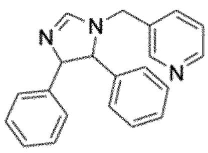

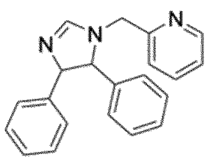

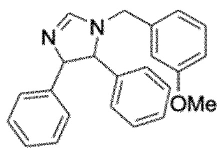

10. Compound of formula (I)

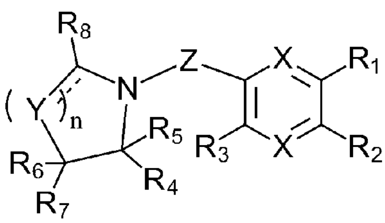

(I)

or a pharmaceutically acceptable salt thereof, wherein
$R_1$ is selected from the group consisting of $R_2$ and

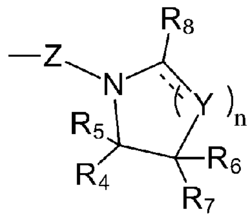

each $R_2$, $R_3$, $R_4$, $R_7$ and $R_8$ are independently selected from H, optionally substituted $C_{1-10}$ alkyl, optionally substituted $C_{2-10}$ alkenyl, optionally substituted $C_{2-10}$ alkynyl, optionally substituted $C_{3-8}$ cycloalkyl, optionally substituted 5-10 membered heteroalicyclic ring, optionally substituted $C_{6-14}$ aryl, optionally substituted 5-14 membered heteroaryl, halogen, cyano, nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$, $-C(=O)R_9$,
$R_5$ and $R_6$ are independently selected from optionally substituted benzyl or optionally substituted phenyl;
wherein one X is N and the other is CH, or one X is $COR_9$ and the other is CH;
each Y is independently selected from $C-R_b$, $C-(R_b)_2$, $N-R_b$ and N;
each Z is independently selected from bivalent $C_{1-4}$ alkyl groups, preferably $-CH_2-$, and $-(CH_2)_2-$; $R_a$ and $R_b$ are independently selected from H, optionally substituted $C_{1-10}$ alkyl, optionally substituted $C_{2-10}$ alkenyl, optionally substituted $C_{2-10}$ alkynyl, optionally substituted $C_{3-8}$ cycloalkyl, optionally substituted 5-10 membered heteroalicyclic ring, optionally substituted $C_{6-14}$ aryl, optionally substituted 5-14 membered heteroaryl, halogen, cyano, nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$, and $-C(=O)R_9$;
$R_9$ and $R_{10}$ are independently selected from H and $C_{1-10}$ alkyl, $C_{2-10}$ alkenyl, $C_{2-10}$ alkynyl, $C_{3-8}$ cycloalkyl, 5-10 membered heteroalicyclic ring, $C_{6-14}$ aryl, 5-14 membered heteroaryl, and combinations thereof;
n is 1 or 2; and
"- - - " indicates a single or double bond,
wherein the substituents are as defined in claim 1;
for use as a pharmaceutical.

**11.** Pharmaceutical composition comprising one or more compounds of formula (I)

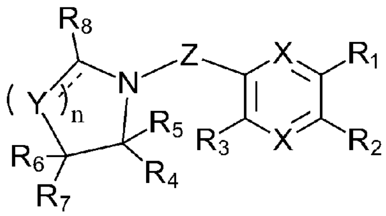

(I)

or a pharmaceutically acceptable salt thereof, wherein
$R_1$ is selected from the group consisting of $R_2$ and

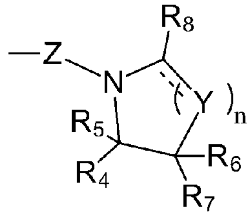

**each** $R_2$, $R_3$, $R_4$, $R_7$ and $R_8$ are independently selected from H, optionally substituted $C_{1-10}$ alkyl, optionally substituted $C_{2-10}$ alkenyl, optionally substituted $C_{2-10}$ alkynyl, optionally substituted $C_{3-8}$ cycloalkyl, optionally substituted 5-10 membered heteroalicyclic ring, optionally substituted $C_{6-14}$ aryl, optionally substituted 5-14 membered heteroaryl, halogen, cyano, nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$, $-C(=O)R_9$,
$R_5$ and $R_6$ are independently selected from optionally substituted benzyl or optionally substituted phenyl;

wherein one X is N and the other is CH, or one X is $COR_9$ and the other is CH;
each Y is independently selected from C-$R_b$, C-$(R_b)_2$, N-$R_b$ and N;
each Z is independently selected from bivalent $C_{1-4}$ alkyl groups, - preferably $-CH_2-$, and $-(CH_2)_2-$; $R_a$ and $R_b$ are independently selected from H, optionally substituted $C_{1-10}$ alkyl, optionally substituted $C_{2-10}$ alkenyl, optionally substituted $C_{2-10}$ alkynyl, optionally substituted $C_{3-8}$ cycloalkyl, optionally substituted 5-10 membered heteroalicyclic ring, optionally substituted $C_{6-14}$ aryl, optionally substituted 5-14 membered heteroaryl, halogen, cyano, nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, - $NR_9C(=O)OR_{10}$, $-COOR_9$, and $-C(=O)R_9$;
$R_9$ and $R_{10}$ are independently selected from H and $C_{1-10}$ alkyl, $C_{2-10}$ alkenyl, $C_{2-10}$ alkynyl, $C_{3-8}$ cycloalkyl, 5-10 membered heteroalicyclic ring, $C_{6-14}$ aryl, 5-14 membered heteroaryl, and combinations thereof;
n is 1 or 2; and
"- - - " indicates a single or double bond;
wherein the substituents are as defined in claim 1; and
a pharmaceutically acceptable excipient and/or carrier.

**12.** The pharmaceutical composition of claim 11, wherein the one or more compounds are selected from the compounds of any one of claims 1 to 9.

**13.** The pharmaceutical composition of claim 11 or 12 further comprising at least one other malaria drug, preferably selected from artemisinin, artesunate, dihydroartemisin, artemotil, lumefantrine, artemether, chloroquine, hydroxychloroquine, amodiaquine, mefloquine, sulfadoxine/pyrimethamine, piperaquine, primaquine, tafenoquine, and ganaplacide.

**14.** The compound of any one of claims 1 to 9 or the pharmaceutical composition of any one of claims 11 to 13 for use in a method for preventing or treating malaria in a subject in need thereof.

## Patentansprüche

**1.** Verbindung mit der Formel (I)

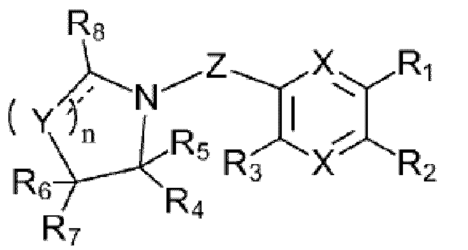

(I)

oder pharmazeutisch verträgliches Salz davon,
wobei
$R_1$ ausgewählt ist aus der Gruppe bestehend aus $R_2$ und

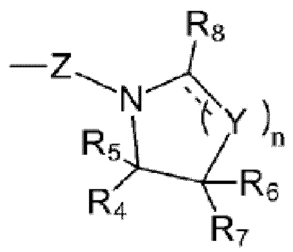

jedes $R_2$, $R_3$, $R_4$, $R_7$ und $R_8$ unabhängig ausgewählt ist aus H, optional substituiertem $C_{1-10}$-Alkyl, optional substituiertem $C_{2-10}$-Alkenyl, optional substituiertem $C_{2-10}$-Alkynyl, optional substituiertem $C_{3-8}$-Cycloalkyl, einem optional substituierten 5-10-gliedrigen heteroalicyclischen Ring, optional substituiertem $C_{6-14}$-Aryl, optional substituiertem 5-14-gliedrigem Heteroaryl, Halogen, Cyano, Nitro, $-OR_9$, $-SR_9$, $-S(O)R_9$, $-S(O)\ _2R_9$, $-NR_9R_{10}$, -C $(=O)NR_9R_{10}$, $-NR_9C(=O)R_{10}$, - OC $(=O)NR_9R_{10}$, $-NR_9C(=O)OR_{10}$, $-COOR_9$, $-C(=O)R_9$,

$R_5$ und $R_6$ unabhängig ausgewählt sind aus optional substituiertem Benzyl oder optional substituiertem Phenyl; wobei ein X N ist, und das andere CH ist, oder ein X $COR_9$ ist, und das andere CH ist;
jedes Y unabhängig ausgewählt ist aus C-$R_b$, C-$(R_b)_2$, N-$R_b$ und N;
jedes Z unabhängig ausgewählt ist aus bivalenten $C_{1-4}$-Alkylgruppen, vorzugsweise $-CH_2-$ und $-(CH_2)_2-$;
$R_a$ und $R_b$ unabhängig ausgewählt sind aus H, optional substituiertem $C_{1-10}$-Alkyl, optional substituiertem $C_{2-10}$-Alkenyl, optional substituiertem $C_{2-10}$-Alkynyl, optional substituiertem $C_{3-8}$-Cycloalkyl, einem optional substituierten 5-10-gliedrigen heteroalicyclischen Ring, optional substituiertem $C_{6-14}$-Aryl, optional substituiertem 5-14-gliedrigem Heteroaryl, Halogen, Cyano, Nitro, $-OR_9$, $-SR_9$, $-S(O)R_9$, $-S(O)_2R^9$, $-NR_9R_{10}$, $- C(=O)NR_9R_{10}$, $-NR_9C(=O)R_{10}$, $-OC(=O)NR_9R_{10}$, $-NR_9C(=O)OR_{10}$, $- COOR_9$ und $-C(=O)R_9$;
$R_9$ und $R_{10}$ unabhängig ausgewählt sind aus H und $C_{1-10}$-Alkyl, $C_{2-10}$-Alkenyl, $C_{2-10}$-Alkynyl, $C_{3-8}$-Cycloalkyl, einem 5-10-gliedrigen heteroalicyclischen Ring, $C_{6-14}$-Aryl, 5-14-gliedrigem Heteroaryl und Kombinationen davon;
n 1 oder 2 ist; und
"- - - " eine Einfach- oder Doppelbindung anzeigt,
wobei die Substituentengruppe(n) im substituierten $C_{1-10}$-Alkyl ein oder mehrere Substituenten sind, individuell ausgewählt aus der Gruppe bestehend aus $C_2$-$C_{10}$-Alkenyl, $C_2$-$C_{10}$-Alkynyl, $C_3$-$C_8$-Cycloalkyl, $C_6$-$C_{14}$-Aryl, 5-14-gliedrigem Heteroaryl, wobei 1 bis 4 Ringatome unabhängig ausgewählt sind aus Stickstoff, Sauerstoff oder Schwefel, einem 5-10-gliedrigen heteroalicyclischen Ring, wobei 1 bis 3 Ringatome unabhängig Stickstoff, Sauerstoff oder Schwefel sind, Hydroxy, $C_1$-$C_{10}$-Alkoxy, $C_3$-$C_8$-Cycloalkoxy, Aryloxy, Mercapto, Alkylthio, Arylthio, Cyano, Halo, Carbonyl, Thiocarbonyl, O-Carbamyl, N-Carbamyl, O-Thiocarbamyl, N-Thiocarbamyl, C-Amido, N-Amido, C-Carboxy, O-Carboxy, Nitro, Sulfinyl, Sulfonyl, Amino, und -N $R^{11}R^{12}$, wobei $R^{11}$ und $R^{12}$ unabhängig ausgewählt sind aus der Gruppe bestehend aus Wasserstoff, $C_1$-$C_4$-Alkyl, $C_3$-$C_8$-Cycloalkyl, $C_{6-14}$-Aryl, Carbonyl, Acetyl, Sulfonyl, Amino und Trifluormethanesulfonyl, oder $R^{11}$ und $R^{12}$, die zusammen mit dem Stickstoffatom, an das sie gebunden sind, zur Bildung eines fünf- oder sechsgliedrigen heteroalicyclischen Rings kombiniert sind; das substituierte $C_{1-10}$-Alkyl umfasst auch Heteroalkyl, wobei mindestens ein Kohlenstoffatom einer gegebenen Alkylgruppe durch ein Heteroatom ersetzt ist, wie zum Beispiel N, O oder S;
wobei die Substituentengruppe (n) im substituierten $C_{2-10}$-Alkenyl wie oben für substituiertes $C_{1-10}$-Alkyl offenbart sind, wobei ein weiterer Substituent Heteroalkenyl ist;
wobei die Substituentengruppe (n) im substituierten $C_{2-10}$-Alkynyl wie oben für substituiertes $C_{1-10}$-Alkyl offenbart sind,
wobei die Substituentengruppe(n) im substituierten $C_{3-8}$-Cycloalkyl wie oben für substituiertes $C_{1-10}$-Alkyl offenbart sind, wobei ein weiterer Substituent $C_{1-10}$-Alkyl ist,
wobei die Substituentengruppe(n) im substituierten 5-10-gliedrigen heteroalicyclischen Ring ein oder mehrere Substituenten ist (sind), unabhängig ausgewählt aus der Gruppe bestehend aus $C_1$-$C_{10}$-Alkyl, $C_2$-$C_{10}$-Alkenyl, $C_2$-$C_{10}$-Alkynyl, $C_3$-$C_8$-Cycloalkyl, $C_6$-$C_{14}$-Aryl, 5-10-gliedrigen Heteroaryl, wobei 1 bis 4 Ringatome unabhängig ausgewählt sind aus Stickstoff, Sauerstoff oder Schwefel, einem 5-10-gliedrigen heteroalicyclischen Ring, wobei 1 bis 3 Ringatome unabhängig Stickstoff, Sauerstoff oder Schwefel sind, Hydroxy, $C_1$-$C_{10}$-Alkoxy, $C_3$-$C_8$-Cycloalkoxy, Aryloxy, Mercapto, Alkylthio, Arylthio, Cyano, Halo, Trihalomethyl, Carbonyl, Thiocarbonyl, O-Carbamyl, N-Carbamyl, O-Thiocarbamyl, N-Thiocarbamyl, C-Amido, N-Amido, C-Carboxy, O-Carboxy, Nitro, Sulfinyl, Sulfonyl, Amino und $-NR^{11}R^{12}$, wobei $R^{11}$ und $R^{12}$ wie oben definiert sind,
wobei die Substituentengruppe(n) im substituierten $C_{6-14}$-Aryl ein oder mehrere Substituenten ist (sind), unabhängig ausgewählt aus der Gruppe bestehend aus $C_1$-$C_{10}$-Alkyl, $C_2$-$C_{10}$-Alkenyl, $C_2$-$C_{10}$-Alkynyl, $C_3$-$C_8$-Cycloalkyl, $C_6$-$C_{14}$-Aryl, 5-14-gliedrigem Heteroaryl, wobei 1 bis 4 Ringatome unabhängig ausgewählt sind aus Stickstoff, Sauerstoff oder Schwefel, einem 5-10-gliedrigen heteroalicyclischen Ring, wobei 1 bis 3 Ringatome unabhängig Stickstoff, Sauerstoff oder Schwefel sind, Hydroxy, $C_1$-$C_{10}$-Alkoxy, $C_3$-$C_8$-Cycloalkoxy, Aryloxy, Mercapto, Alkylthio, Arylthio, Cyano, Halo, Trihalomethyl, Carbonyl, Thiocarbonyl, O-Carbamyl, N-Carbamyl, O-Thiocarbamyl, N-Thiocarbamyl, C-Amido, N-Amido, C-Carboxy, O-Carboxy, Nitro, Sulfinyl, Sulfonyl, Amino, und $-NR^{11}R^{12}$, wobei $R^{11}$ und $R^{12}$ wie oben definiert sind,
wobei die Substituentengruppe(n) im substituierten 5-14-gliedrigen Heteroaryl ein oder mehrere Substituenten ist (sind), unabhängig ausgewählt aus der Gruppe bestehend aus $C_1$-$C_{10}$-Alkyl, $C_2$-$C_{10}$-Alkenyl, $C_2$-$C_{10}$-Alkynyl, $C_3$-$C_8$-Cycloalkyl, $C_6$-$C_{14}$-Aryl, 5-14-gliedrigem Heteroaryl, wobei 1 bis 4 Ringatome unabhängig ausgewählt sind aus Stickstoff, Sauerstoff oder Schwefel, einem 5-10-gliedrigen heteroalicyclischen Ring, wobei 1 bis 3 Ringatome unabhängig Stickstoff, Sauerstoff oder Schwefel sind, Hydroxy, $C_1$-$C_{10}$-Alkoxy, $C_3$-$C_8$-Cycloalkoxy, Aryloxy, Mercapto, Alkylthio, Arylthio, Cyano, Halo, Trihalomethyl, Carbonyl, Thiocarbonyl, O-Carbamyl, N-Carbamyl, O-Thiocarbamyl, N-Thiocarbamyl, C-Amido, N-Amido, C-Carboxy, O-Carboxy, Nitro, Sulfinyl, Sulfonyl, Amino, und $-NR^{11}R^{12}$, wobei $R^{11}$ und $R^{12}$ wie oben definiert sind.

2. Verbindung nach Anspruch 1, wobei Z $-CH_2-$ ist.

**3.** Verbindung nach Anspruch 1 oder 2, wobei n 1 ist, Y N ist, und "---" eine Doppelbindung ist.

**4.** Verbindung nach Anspruch 1 oder 2, wobei n 1 ist, Y $CR_b$ ist, vorzugsweise CH, und "---" eine Einfachbindung ist.

**5.** Verbindung nach einem der Ansprüche 1 bis 4, wobei $R_1$

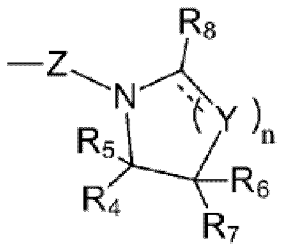

ist.

**6.** Verbindung nach Anspruch 5, wobei die Verbindung insofern symmetrisch ist, dass sowohl Z, als auch $(Y)_n$, als auch $R_4$, als auch $R_5$, als auch $R_6$, als auch $R_7$ identisch sind.

**7.** Verbindung nach einem der Ansprüche 1 bis 6, wobei sowohl $R_5$ als auch $R_6$ unsubstituiertes Phenyl sind, und sowohl $R_4$ als auch $R_7$ Wasserstoff sind.

**8.** Verbindung nach einem der Ansprüche 1 bis 7, wobei $R_8$ H ist.

**9.** Verbindung nach einem der Ansprüche 1 bis 8, wobei die Verbindung aus einer der folgenden Verbindungen ausgewählt ist:

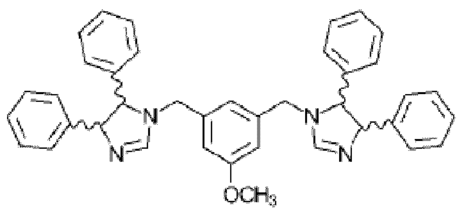

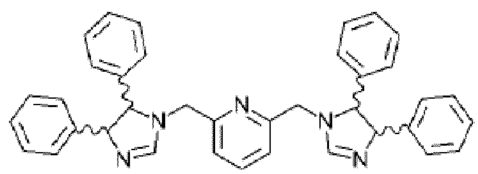

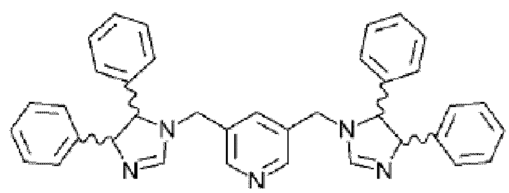

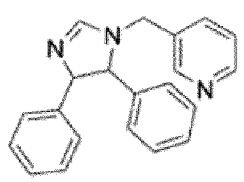

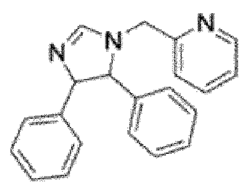

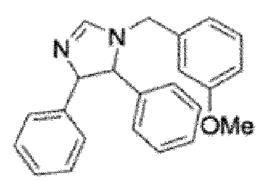

.

**10.** Verbindung mit der Formel (I)

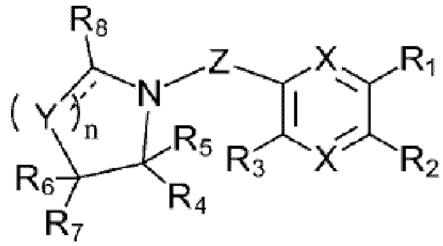

(I)

oder pharmazeutisch verträgliches Salz davon,
wobei
$R_1$ ausgewählt ist aus der Gruppe bestehend aus $R_2$ und

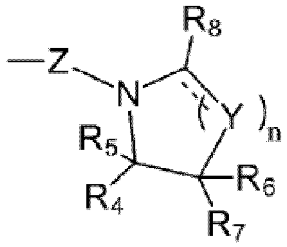

jedes $R_2$, $R_3$, $R_4$, $R_7$ und $R_8$ unabhängig ausgewählt ist aus H, optional substituiertem $C_{1-10}$-Alkyl, optional substituiertem $C_{2-10}$-Alkenyl, optional substituiertem $C_{2-10}$-Alkynyl, optional substituiertem $C_{3-8}$-Cycloalkyl, einem optional substituierten 5-10-gliedrigen heteroalicyclischen Ring, optional substituiertem $C_{6-14}$-Aryl, optional substituiertem 5-14-gliedrigem Heteroaryl, Halogen, Cyano, Nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, - $OC(=O)NR_9R_{10}$, $-NR_9C(=O)OR_{10}$, $-COOR_9$, $-C(=O)R_9$,
$R_5$ und $R_6$ unabhängig ausgewählt sind aus optional substituiertem Benzyl oder optional substituiertem Phenyl;
wobei ein X N ist, und das andere CH ist, oder ein X $COR_9$ ist, und das andere CH ist;
jedes Y unabhängig ausgewählt ist aus $C-R_b$, $C-(R_b)_2$, $N-R_b$ und N;
jedes Z unabhängig ausgewählt ist aus bivalenten $C_{1-4}$-Alkylgruppen, vorzugsweise $-CH_2-$ und - $(CH_2)_2-$;
$R_a$ und $R_b$ unabhängig ausgewählt sind aus H, optional substituiertem $C_{1-10}$-Alkyl, optional substituiertem $C_{2-10}$-Alkenyl, optional substituiertem $C_{2-10}$-Alkynyl, optional substituiertem $C_{3-8}$-Cycloalkyl, einem optional substituierten 5-10-gliedrigen heteroalicyclischen Ring, optional substituiertem $C_{6-14}$-Aryl, optional substituiertem 5-14-gliedrigem Heteroaryl, Halogen, Cyano, Nitro, $-OR_9$, $-SR_9$, $-S(O)R_9$, $-S(O)_2R^9$, $-NR_9R_{10}$, - $C(=O)NR_9R_{10}$, $-NR_9C(=O)R_{10}$, $-OC(=O)NR_9R_{10}$, $-NR_9C(=O)OR_{10}$, - $COOR_9$ und $-C(=O)R_9$;
$R_9$ und $R_{10}$ unabhängig ausgewählt sind aus H und $C_{1-10}$-Alkyl, $C_{2-10}$-Alkenyl, $C_{2-10}$-Alkynyl, $C_{3-8}$-Cycloalkyl, einem 5-10-gliedrigen heteroalicyclischen Ring, $C_{6-14}$-Aryl, 5-14-gliedrigem Heteroaryl und Kombinationen davon;
n 1 oder 2 ist; und
"---" eine Einfach- oder Doppelbindung anzeigt,
wobei die Substituenten wie in Anspruch 1 definiert sind;
zur Verwendung als Pharmazeutikum.

**11.** Pharmazeutische Zusammensetzung, umfassend eine oder

mehrere Verbindungen mit der Formel (I)

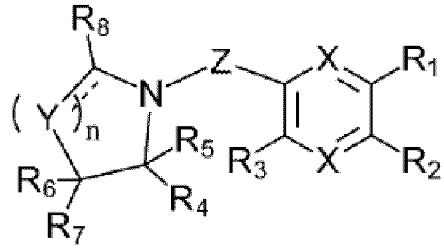

(I)

(I)

oder pharmazeutisch verträgliches Salz davon,
wobei
$R_1$ ausgewählt ist aus der Gruppe bestehend aus $R_2$ und

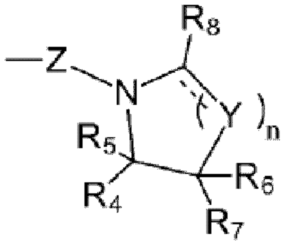

jedes $R_2$, $R_3$, $R_4$, $R_7$ und $R_8$ unabhängig ausgewählt ist aus H, optional substituiertem $C_{1-10}$-Alkyl, optional substituiertem $C_{2-10}$-Alkenyl, optional substituiertem $C_{2-10}$-Alkynyl, optional substituiertem $C_{3-8}$-Cycloalkyl, einem optional substituierten 5-10-gliedrigen heteroalicyclischen Ring, optional substituiertem $C_{6-14}$-Aryl, optional substituiertem 5-14-gliedrigem Heteroaryl, Halogen, Cyano, Nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $- OC(=O)NR_9R_{10}$, $-NR_9C(=O)OR_{10}$, $-COOR_3$, $-C (=O)R_9$,
$R_5$ und $R_6$ unabhängig ausgewählt sind aus optional substituiertem Benzyl oder optional substituiertem Phenyl;
wobei ein X N ist, und das andere CH ist, oder ein X $COR_9$ ist, und das andere CH ist;
jedes Y unabhängig ausgewählt ist aus $C-R_b$, $C-(R_b)_2$, $N-R_b$ und N;
jedes Z unabhängig ausgewählt ist aus bivalenten $C_{1-4}$-Alkylgruppen, vorzugsweise $-CH_2-$ und -(CH2)2-;
$R_a$ und $R_b$ unabhängig ausgewählt sind aus H, optional substituiertem $C_{1-10}$-Alkyl, optional substituiertem $C_{2-10}$-Alkenyl, optional substituiertem $C_{2-10}$-Alkynyl, optional substituiertem $C_{3-8}$-Cycloalkyl, einem optional substituierten 5-10-gliedrigen heteroalicyclischen Ring, optional substituiertem $C_{6-14}$-Aryl, optional substituiertem 5-14-gliedrigem Heteroaryl, Halogen, Cyano, Nitro, $-OR_9$, $-SR_9$, $-S(O)R_9$, $-S(O)_2R_9$, $-NR_9R_{10}$, $- C (=O) NR_9R_{10}$, $-NR_9C(=O)R_{10}$, $-OC (=O)NR_9R_{10}$, $-NR_9C(=O)OR_{10}$, $- COOR_9$ und $-C(=O)R_9$;
$R_9$ und $R_{10}$ unabhängig ausgewählt sind aus H und $C_{1-10}$-Alkyl, $C_{2-10}$-Alkenyl, $C_{2-10}$-Alkynyl, $C_{3-8}$-Cycloalkyl, einem 5-10-gliedrigen heteroalicyclischen Ring, $C_{6-14}$-Aryl, 5-14-gliedrigem Heteroaryl und Kombinationen davon;
n 1 oder 2 ist; und
"- - -" eine Einfach- oder Doppelbindung anzeigt;
wobei die Substituenten wie in Anspruch 1 definiert sind; und
einen pharmazeutisch verträglichen Exzipienten und/oder Träger.

**12.** Pharmazeutische Zusammensetzung nach Anspruch 11, wobei die eine oder die mehreren Verbindungen ausgewählt sind aus den Verbindungen nach einem der Ansprüche 1 bis 9.

**13.** Pharmazeutische Zusammensetzung nach Anspruch 11 oder 12, ferner umfassend mindestens ein weiteres Malariamedikament, vorzugsweise ausgewählt aus Artemisinin, Artesunat, Dihydroartemisin, Artemotil, Lumefantrin, Artemether, Chloroquin, Hydroxychloroquin, Amodiaquin, Mefloquin, Sulfadoxin/Pyrimethamin, Piperaquin, Primaquin, Tafenoquin und Gganaplacid.

**14.** Verbindung nach einem der Ansprüche 1 bis 9 oder pharmazeutische Zusammensetzung nach einem der Ansprüche 11 bis 13 zur Verwendung in einem Verfahren zur Prävention oder Behandlung von Malaria bei einer dies benötigenden Person.

## Revendications

**1.** Composé de formule (I)

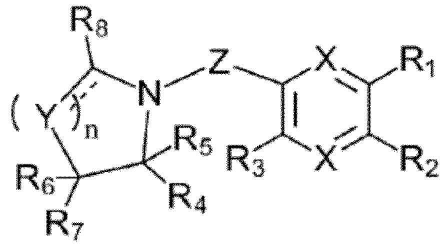

(I)

ou un sel pharmaceutiquement acceptable de celui-ci, dans lequel
$R_1$ est choisi dans le groupe constitué par $R_2$ et

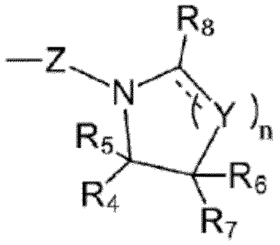

chaque $R_2$, $R_3$, $R_4$, $R_7$ et $R_8$ sont indépendamment choisis parmi H, un groupe alkyle en $C_{1-10}$ éventuellement substitué, un groupe alcényle en $C_{2-10}$ éventuellement substitué, un groupe alcynyle en $C_{2-10}$ éventuellement substitué, un groupe cycloalkyle en $C_{3-8}$ éventuellement substitué, un cycle hétéroalicyclique de 5 à 10 chaînons éventuellement substitué, un groupe aryle en $C_{6-14}$ éventuellement substitué, un groupe hétéroaryle de 5 à 14 chaînons éventuellement substitué, un atome d'halogène, un groupe cyano, un groupe nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$, $-C(=O)R_9$,
$R_5$ et $R_6$ sont indépendamment choisi parmi un groupe benzyle éventuellement substitué ou un groupe phényle éventuellement substitué ;
dans lequel un X est N et l'autre est CH, ou un X est $COR_9$ et l'autre est CH ;
chaque Y est indépendamment choisi parmi $C-R_b$, $C-(R_b)_2$, $N-R_b$ et N ;
chaque Z est indépendamment choisi parmi les groupes alkyle en $C_{1-4}$ bivalents, de préférence $-CH_2-$ et $-(CH_2)_2-$ ;
$R_a$ et $R_b$ sont indépendamment choisis parmi H, un groupe alkyle en $C_{1-10}$ éventuellement substitué, un groupe alcényle en $C_{2-10}$ éventuellement substitué, un groupe alcynyle en $C_{2-10}$ éventuellement substitué, un groupe cycloalkyle en $C_{3-8}$ éventuellement substitué, un cycle hétéroalicyclique de 5 à 10 chaînons éventuellement substitué, un groupe aryle en $C_{6-14}$ éventuellement substitué, un groupe hétéroaryle de 5 à 14 chaînons éventuellement substitué, un atome d'halogène, un groupe cyano, un groupe nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$ et $-C(=O)R_9$ ;
$R_9$ et $R_{10}$ sont indépendamment choisis parmi H et un groupe alkyle en $C_{1-10}$, un groupe alcényle en $C_{2-10}$, un groupe alcynyle en $C_{2-10}$, un groupe cycloalkyle en $C_{3-8}$, un cycle hétéroalicyclique de 5 à 10 chaînons, un groupe aryle en $C_{6-14}$, un groupe hétéroaryle de 5 à 14 chaînons et des combinaisons de ceux-ci ;
n est 1 ou 2 ; et
« - - - » indique une liaison simple ou double,
dans lequel le(s) groupe(s) substituant(s) dans le groupe alkyle en $C_{1-10}$ substitué sont un ou plusieurs substituants, individuellement choisis dans le groupe constitué par un groupe alcényle en $C_2$ à $C_{10}$, un groupe alcynyle en $C_2$ à $C_{10}$, un groupe cycloalkyle en $C_3$ à $C_8$, un groupe aryle en $C_6$ à $C_{14}$, un groupe hétéroaryle de 5 à 14 chaînons dans lequel 1 à 4 atomes du cycle sont indépendamment choisis parmi un atome d'azote, un atome d'oxygène ou un atome de soufre, un groupe hétéroalicyclique de 5 à 10 chaînons dans lequel 1 à 3 atomes du cycle sont indépendamment un atome d'azote, un atome d'oxygène ou un atome de soufre, un groupe hydroxy, un groupe alcoxy en $C_1$ à $C_{10}$, un groupe cycloalcoxy en $C_3$ à $C_8$, un groupe aryloxy, un groupe mercapto, un groupe alkylthio, un groupe arylthio, un groupe cyano, un atome d'halogène, un groupe carbonyle, un groupe thiocarbonyle, un groupe O-carbamyle, un groupe N-carbamyle, un groupe O-thiocarbamyle, un groupe N-thiocarbamyle, un groupe C-amido, un groupe N-amido, un groupe C-carboxy, un groupe O-carboxy, un groupe nitro, un groupe sulfinyle, un groupe sulfonyle, un groupe amino et $-NR^{11}R^{12}$ où $R^{11}$ et $R^{12}$ sont indépendamment choisis dans le groupe constitué par un atome d'hydrogène, un groupe alkyle en $C_1$ à $C_4$, un groupe cycloalkyle en $C_3$ à $C_8$, un groupe aryle en $C_6$ à $C_{14}$, un groupe carbonyle, un groupe acétyle, un groupe sulfonyle, un groupe

amino et un groupe trifluorométhanesulfonyle, ou $R^{11}$ et $R^{12}$, conjointement avec l'atome d'azote auquel ils sont liés, se combinent pour former un cycle hétéroalicyclique de cinq ou six chaînons ; un groupe alkyle en $C_{1-10}$ substitué comprend également un groupe hétéroalkyle où au moins un atomes de carbone d'un groupe alkyle donné est remplacé par un hétéroatome, tel que N, O ou S ;

dans lequel le(s) groupe(s) substituant(s) dans le groupe alcényle en $C_{2-10}$ substitué sont tels que divulgués ci-dessus pour le groupe alkyle en $C_{1-10}$ substitué avec un substituant supplémentaire qui est un groupe hétéroalcényle ;

dans lequel le(s) groupe(s) substituant(s) dans le groupe alcynyle en $C_{2-10}$ substitué sont tels que divulgués ci-dessus pour le groupe alkyle en $C_{1-10}$ substitué,

dans lequel le(s) groupe(s) substituant(s) dans le groupe cycloalkyle en $C_{3-8}$ substitué sont tels que divulgués ci-dessus pour le groupe alkyle en $C_{1-10}$ substitué avec un substituant supplémentaire qui est un groupe alkyle en $C_{1-10}$,

dans lequel le(s) groupe(s) substituant(s) dans le cycle hétéroalicyclique de 5 à 10 chaînons substitué sont un ou plusieurs substituants, indépendamment choisis dans le groupe constitué par un groupe alkyle en $C_1$ à $C_{10}$, un groupe alcényle en $C_2$ à $C_{10}$, un groupe alcynyle en $C_2$ à $C_{10}$, un groupe cycloalkyle en $C_3$ à $C_8$, un groupe aryle en $C_6$ à $C_{14}$, un groupe hétéroaryle de 5 à 10 chaînons dans lequel 1 à 4 atomes du cycle sont indépendamment choisis parmi un atome d'azote, un atome d'oxygène ou un atome de soufre, un groupe hétéroalicyclique de 5 à 10 chaînons dans lequel 1 à 3 atomes du cycle sont indépendamment un atome d'azote, un atome d'oxygène ou un atome de soufre, un groupe hydroxy, un groupe alcoxy en $C_1$ à $C_{10}$, un groupe cycloalcoxy en $C_3$ à $C_8$, un groupe aryloxy, un groupe mercapto, un groupe alkylthio, un groupe arylthio, un groupe cyano, un atome d'halogène un groupe carbonyle, un groupe thiocarbonyle, un groupe O-carbamyle, un groupe N-carbamyle, un groupe O-thiocarbamyle, un groupe N-thiocarbamyle, un groupe C-amido, un groupe N-amido, un groupe C-carboxy, un groupe O-carboxy, un groupe nitro, un groupe sulfinyle, un groupe sulfonyle, un groupe amino et $-NR^{11}R^{12}$ avec $R^{11}$ et $R^{12}$ qui sont tels que définis ci-dessus,

dans lequel le(s) groupe(s) substituant(s) dans le groupe aryle en $C_6$ à $C_{14}$ substitué sont un ou plusieurs substituants, indépendamment choisis dans le groupe constitué par un groupe alkyle en $C_1$ à $C_{10}$, un groupe alcényle en $C_2$ à $C_{10}$, un groupe alcynyle en $C_2$ à $C_{10}$, un groupe cycloalkyle en $C_3$ à $C_8$, un groupe aryle en $C_6$ à $C_{14}$, un groupe hétéroaryle de 5 à 14 chaînons dans lequel 1 à 4 atomes du cycle sont indépendamment choisis parmi un atome d'azote, un atome d'oxygène ou un atome de soufre, un groupe hétéroalicyclique de 5 à 10 chaînons dans lequel 1 à 3 atomes du cycle sont indépendamment un atome d'azote, un atome d'oxygène ou un atome de soufre, un groupe hydroxy, un groupe alcoxy en $C_1$ à $C_{10}$, un groupe cycloalcoxy en $C_3$ à $C_8$, un groupe aryloxy, un groupe mercapto, un groupe alkylthio, un groupe arylthio, un groupe cyano, un atome d'halogène un groupe carbonyle, un groupe thiocarbonyle, un groupe O-carbamyle, un groupe N-carbamyle, un groupe O-thiocarbamyle, un groupe N-thiocarbamyle, un groupe C-amido, un groupe N-amido, un groupe C-carboxy, un groupe O-carboxy, un groupe nitro, un groupe sulfinyle, un groupe sulfonyle, un groupe amino et $-NR^{11}R^{12}$ avec $R^{11}$ et $R^{12}$ qui sont tels que définis ci-dessus,

dans lequel le(s) groupe(s) substituant(s) dans le groupe hétéroaryle de 5 à 14 chaînons substitué sont un ou plusieurs substituants, indépendamment choisis dans le groupe constitué par un groupe alkyle en $C_1$ à $C_{10}$, un groupe alcényle en $C_2$ à $C_{10}$, un groupe alcynyle en $C_2$ à $C_{10}$, un groupe cycloalkyle en $C_3$ à $C_8$, un groupe aryle en $C_6$ à $C_{14}$, un groupe hétéroaryle de 5 à 14 chaînons dans lequel 1 à 4 atomes du cycle sont indépendamment choisis parmi un atome d'azote, un atome d'oxygène ou un atome de soufre, un groupe hétéroalicyclique de 5 à 10 chaînons dans lequel 1 à 3 atomes du cycle sont indépendamment un atome d'azote, un atome d'oxygène ou un atome de soufre, un groupe hydroxy, un groupe alcoxy en $C_1$ à $C_{10}$, un groupe cycloalcoxy en $C_3$ à $C_8$, un groupe aryloxy, un groupe mercapto, un groupe alkylthio, un groupe arylthio, un groupe cyano, un atome d'halogène un groupe carbonyle, un groupe thiocarbonyle, un groupe O-carbamyle, un groupe N-carbamyle, un groupe O-thiocarbamyle, un groupe N-thiocarbamyle, un groupe C-amido, un groupe N-amido, un groupe C-carboxy, un groupe O-carboxy, un groupe nitro, un groupe sulfinyle, un groupe sulfonyle, un groupe amino et $-NR^{11}R^{12}$ avec $R^{11}$ et $R^{12}$ qui sont tels que définis ci-dessus.

**2.** Composé selon la revendication 1, dans lequel Z est $-CH_2-$.

**3.** Composé selon la revendication 1 ou 2, dans lequel n est 1, Y est N et « - - - » est une double liaison.

**4.** Composé selon la revendication 1 ou 2, dans lequel n est 1, Y est $CR_b$, de préférence CH, et « - - - » est une liaison simple.

**5.** Composé selon l'une quelconque des revendications 1 à 4, dans lequel $R_1$ est

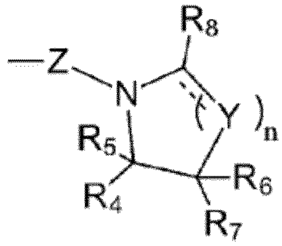

.

6. Composé selon la revendication 5, dans lequel le composé est symétrique en ce que les deux Z, les deux $(Y)_n$, les deux $R_4$, les deux $R_5$, les deux $R_6$ et les deux $R_7$ sont identiques.

7. Composé selon l'une quelconque des revendications 1 à 6, dans lequel les deux $R_5$ et $R_6$ sont un groupe phényle non substitué, et $R_4$ et $R_7$ sont tous deux un atome d'hydrogène.

8. Composé selon l'une quelconque des revendications 1 à 7, dans lequel $R_8$ est H.

9. Composé selon l'une quelconque des revendications 1 à 8, dans lequel le composé est choisi parmi l'un quelconque des composés suivants :

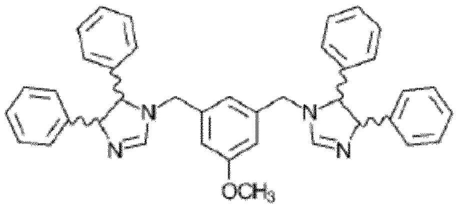

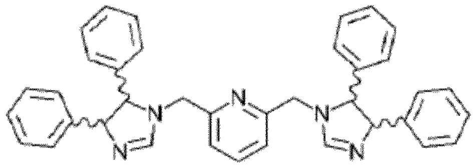

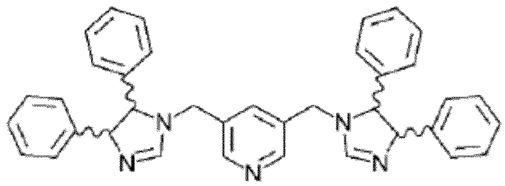

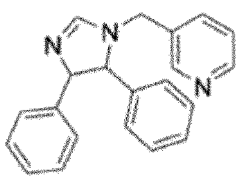

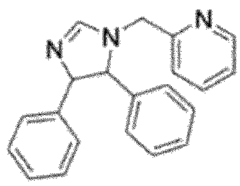

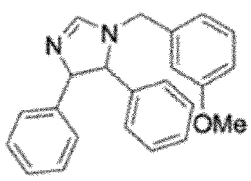

.

10. Composé de formule (I)

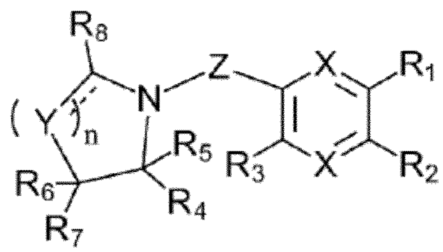

(I)

ou un sel pharmaceutiquement acceptable de celui-ci, dans lequel
$R_1$ est choisi dans le groupe constitué par $R_2$ et

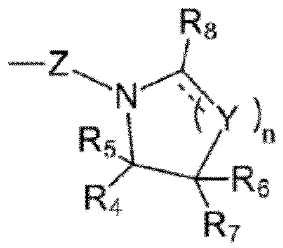

chaque $R_2$, $R_3$, $R_4$, $R_7$ et $R_8$ sont indépendamment choisis parmi H, un groupe alkyle en $C_{1-10}$ éventuellement substitué, un groupe alcényle en $C_{2-10}$ éventuellement substitué, un groupe alcynyle en $C_{2-10}$ éventuellement substitué, un groupe cycloalkyle en $C_{3-8}$ éventuellement substitué, un cycle hétéroalicyclique de 5 à 10 chaînons éventuellement substitué, un groupe aryle en $C_{6-14}$ éventuellement substitué, un groupe hétéroaryle de 5 à 14 chaînons éventuellement substitué, un atome d'halogène, un groupe cyano, un groupe nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$, $-C(=O)R_9$,

$R_5$ et $R_6$ sont indépendamment choisi parmi un groupe benzyle éventuellement substitué ou un groupe phényle éventuellement substitué ;

dans lequel un X est N et l'autre est CH, ou un X est $COR_9$ et l'autre est CH ;

chaque Y est indépendamment choisi parmi $C-R_b$, $C-(R_b)_2$, $N-R_b$ et N ;

chaque Z est indépendamment choisi parmi les groupes alkyle en $C_{1-4}$ bivalents, de préférence $-CH_2-$ et $-(CH_2)_2-$ ;

$R_a$ et $R_b$ sont indépendamment choisis parmi H, un groupe alkyle en $C_{1-10}$ éventuellement substitué, un groupe alcényle en $C_{2-10}$ éventuellement substitué, un groupe alcynyle en $C_{2-10}$ éventuellement substitué, un groupe cycloalkyle en $C_{3-8}$ éventuellement substitué, un cycle hétéroalicyclique de 5 à 10 chaînons éventuellement substitué, un groupe aryle en $C_{6-14}$ éventuellement substitué, un groupe hétéroaryle de 5 à 14 chaînons éventuellement substitué, un atome d'halogène, un groupe cyano, un groupe nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$ et $-C(=O)R_9$ ;

$R_9$ et $R_{10}$ sont indépendamment choisis parmi H et un groupe alkyle en $C_{1-10}$, un groupe alcényle en $C_{2-10}$, un groupe alcynyle en $C_{2-10}$, un groupe cycloalkyle en $C_{3-8}$, un cycle hétéroalicyclique de 5 à 10 chaînons, un groupe aryle en $C_{6-14}$, un groupe hétéroaryle de 5 à 14 chaînons et des combinaisons de ceux-ci ;

n est 1 ou 2 ; et

« - - - » indique une liaison simple ou double,

dans lequel les substituants sont tels que définis dans la revendication 1 ;

pour une utilisation comme produit pharmaceutique.

**11.** Composition pharmaceutique comprenant un ou plusieurs composés de formule (I)

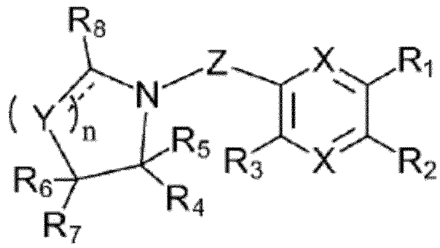

(I)

ou un sel pharmaceutiquement acceptable de celui-ci, dans lequel

$R_1$ est choisi dans le groupe constitué par $R_2$ et

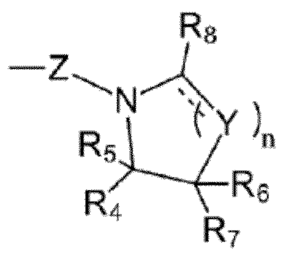

chaque $R_2$, $R_3$, $R_4$, $R_7$ et $R_8$ sont indépendamment choisis parmi H, un groupe alkyle en $C_{1-10}$ éventuellement substitué, un groupe alcényle en $C_{2-10}$ éventuellement substitué, un groupe alcynyle en $C_{2-10}$ éventuellement substitué, un groupe cycloalkyle en $C_{3-8}$ éventuellement substitué, un cycle hétéroalicyclique de 5 à 10 chaînons éventuellement substitué, un groupe aryle en $C_{6-14}$ éventuellement substitué, un groupe hétéroaryle de 5 à 14 chaînons éventuellement substitué, un atome d'halogène, un groupe cyano, un groupe nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$, $-C(=O)R_9$,

$R_5$ et $R_6$ sont indépendamment choisi parmi un groupe benzyle éventuellement substitué ou un groupe phényle éventuellement substitué ;

dans lequel un X est N et l'autre est CH, ou un X est $COR_9$ et l'autre est CH ;

chaque Y est indépendamment choisi parmi $C-R_b$, $C-(R_b)_2$, $N-R_b$ et N ;

chaque Z est indépendamment choisi parmi les groupes alkyle en $C_{1-4}$ bivalents, de préférence $-CH_2-$ et $-(CH_2)_2-$ ;

$R_a$ et $R_b$ sont indépendamment choisis parmi H, un groupe alkyle en $C_{1-10}$ éventuellement substitué, un groupe alcényle en $C_{2-10}$ éventuellement substitué, un groupe alcynyle en $C_{2-10}$ éventuellement substitué, un groupe cycloalkyle en $C_{3-8}$ éventuellement substitué, un cycle hétéroalicyclique de 5 à 10 chaînons éventuellement substitué, un groupe aryle en $C_{6-14}$ éventuellement substitué, un groupe hétéroaryle de 5 à 14 chaînons éventuellement substitué, un atome d'halogène, un groupe cyano, un groupe nitro, $-OR_9$, $-SR_9$, $-S(O)R_9$, $-S(O)_2R_9$, $-NR_9R_{10}$, $-C(=O)NR_9R_{10}$, $-NR_9C(=O)R_{10}$, $-OC(=O)NR_9R_{10}$, $-NR_9C(=O)OR_{10}$, $-COOR_9$ et $-C(=O)R_9$ ;

$R_9$ et $R_{10}$ sont indépendamment choisis parmi H et un groupe alkyle en $C_{1-10}$, un groupe alcényle en $C_{2-10}$, un groupe alcynyle en $C_{2-10}$, un groupe cycloalkyle en $C_{3-8}$, un cycle hétéroalicyclique de 5 à 10 chaînons, un groupe aryle en $C_{6-14}$, un groupe hétéroaryle de 5 à 14 chaînons et des combinaisons de ceux-ci ;

n est 1 ou 2 ; et

« - - - » indique une liaison simple ou double,

dans lequel les substituants sont tels que définis dans la revendication 1 ; et

un excipient et/ou un véhicule pharmaceutiquement acceptable.

**12.** Composition pharmaceutique selon la revendication 11, dans laquelle le ou les composés sont choisis parmi les composés selon l'une quelconque des revendications 1 à 9.

**13.** Composition pharmaceutique selon la revendication 11 ou 12 comprenant en outre au moins un autre médicament antipaludique, de préférence choisi parmi l'artémisinine, l'artésunate, la dihydroartémisine, l'artémotil, la luméfantrine, l'artéméther, la chloroquine, l'hydroxychloroquine, l'amodiaquine, la méfloquine, la sulfadoxine/pyriméthamine, la pipéraquine, la primaquine, la tafénoquine et le ganaplacide.

**14.** Composé selon l'une quelconque des revendications 1 à 9 ou composition pharmaceutique selon l'une quelconque des revendications 11 à 13 pour une utilisation dans une méthode de prévention ou de traitement du paludisme chez un sujet en ayant besoin.

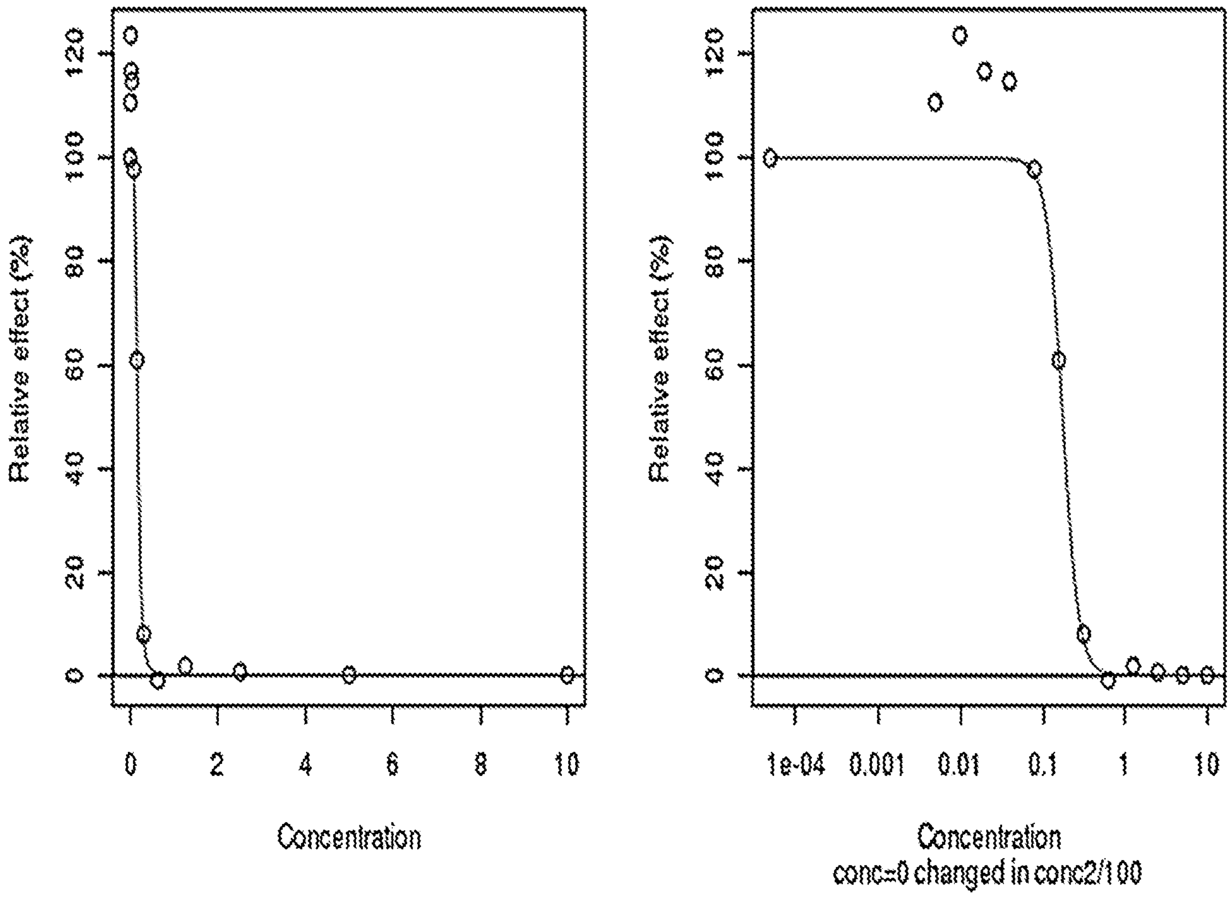


ICn Estimations

| | Estimation | Standard error | Lower CI95% | Upper CI95% | Ratio CI |
|---|---|---|---|---|---|
| IC50 | 0.17 | 0.02 | 0.14 | 0.21 | 1.56 |
| IC90 | 0.28 | 0.02 | 0.24 | 0.33 | 1.4 |
| IC99 | 0.49 | 0.03 | 0.43 | 0.55 | 1.28 |
| γ | 4.43 | 2.18 | -0.37 | 9.24 | |

**Figure 1**

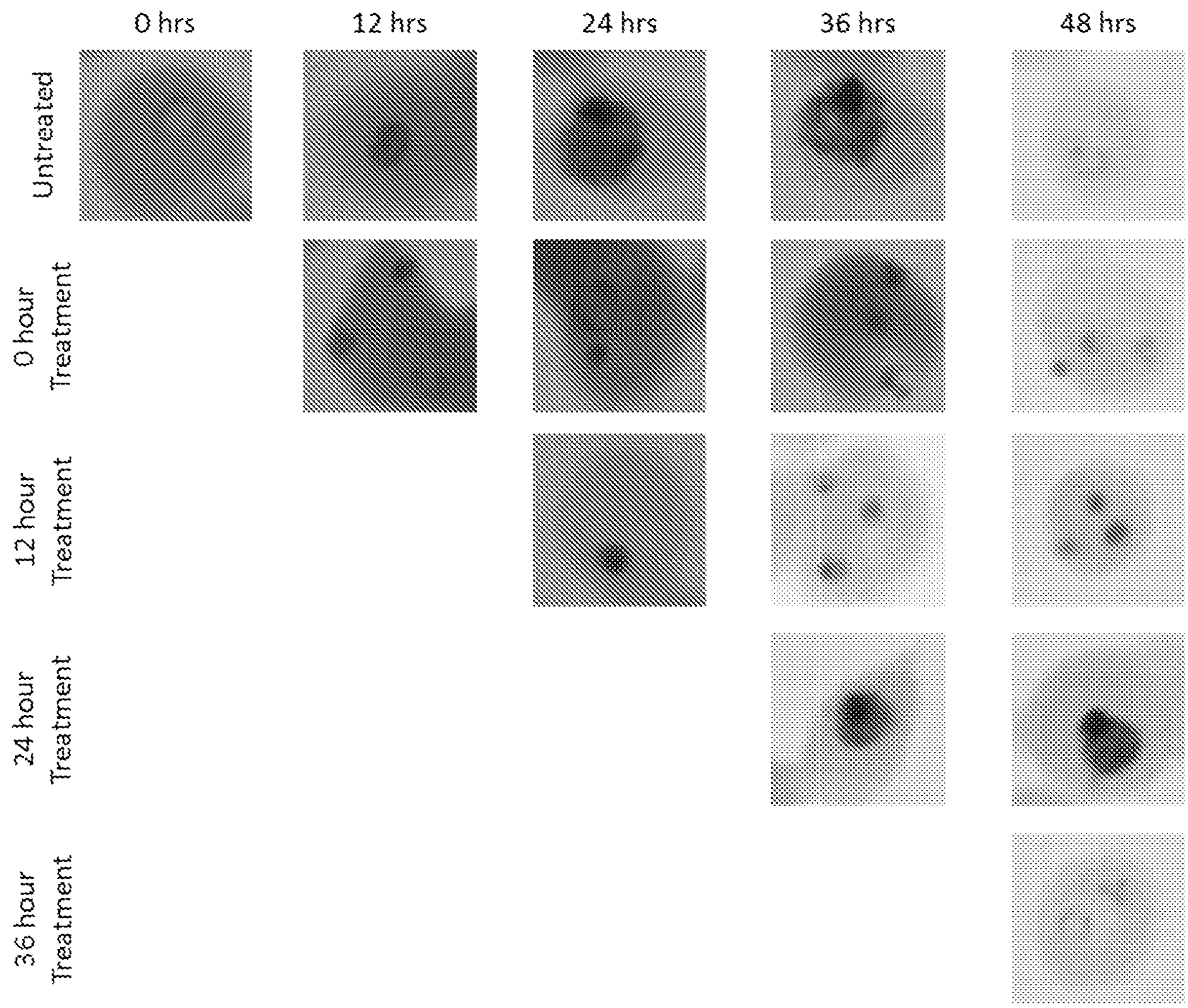
0 hrs
12 hrs
24 hrs
36 hrs
48 hrs
Untreated
0 hour Treatment
12 hour Treatment
24 hour Treatment
36 hour Treatment

Figure 2

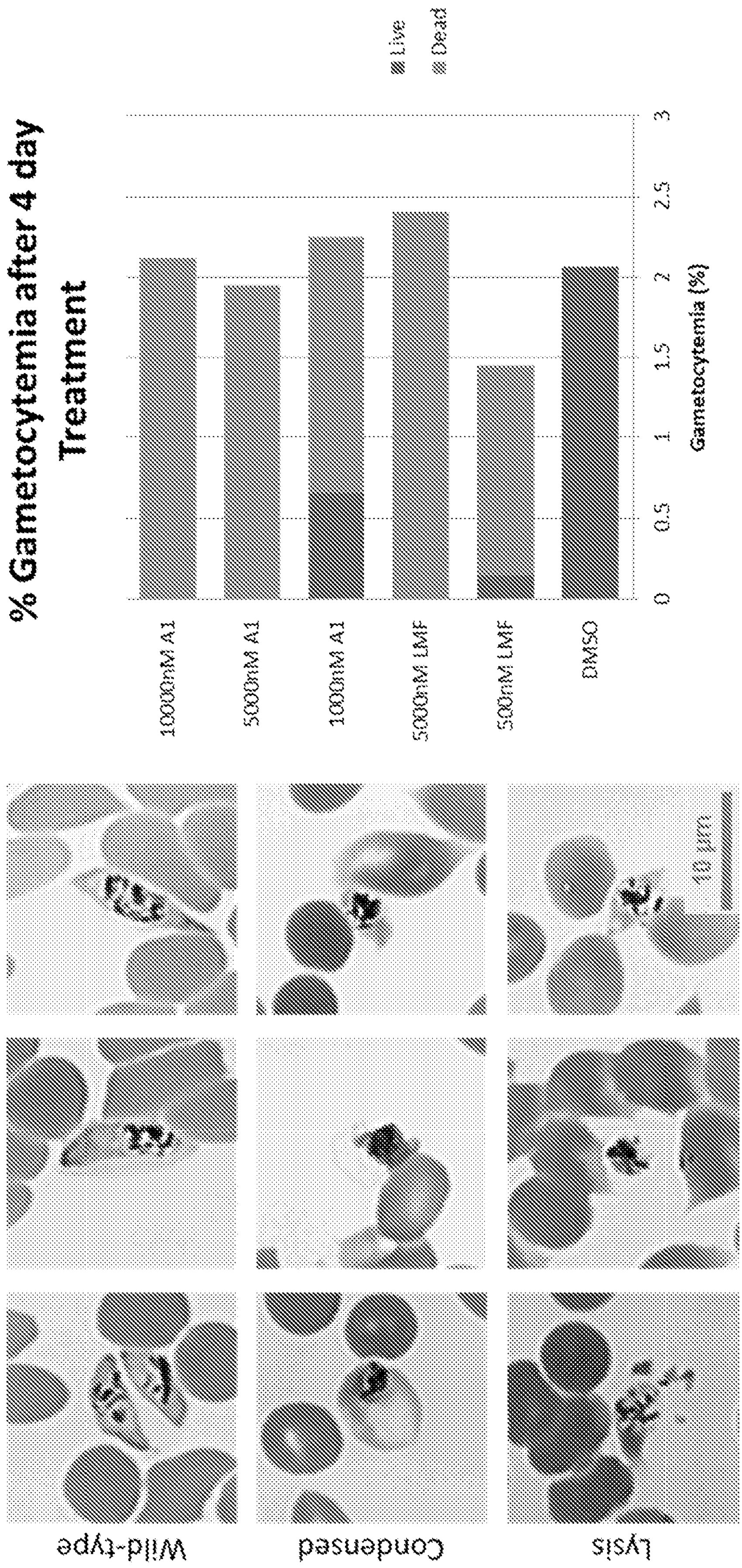
% Gametocytemia after 4 day Treatment
1000nM A1
5000nM A1
1000nM A1
5000nM LMF
500nM LMF
DMSO
0
0.5
1
1.5
2
2.5
3
Gametocytemia (%)
Live
Dead
Wild-type
Condensed
Lysis
10 µm

Figure 3

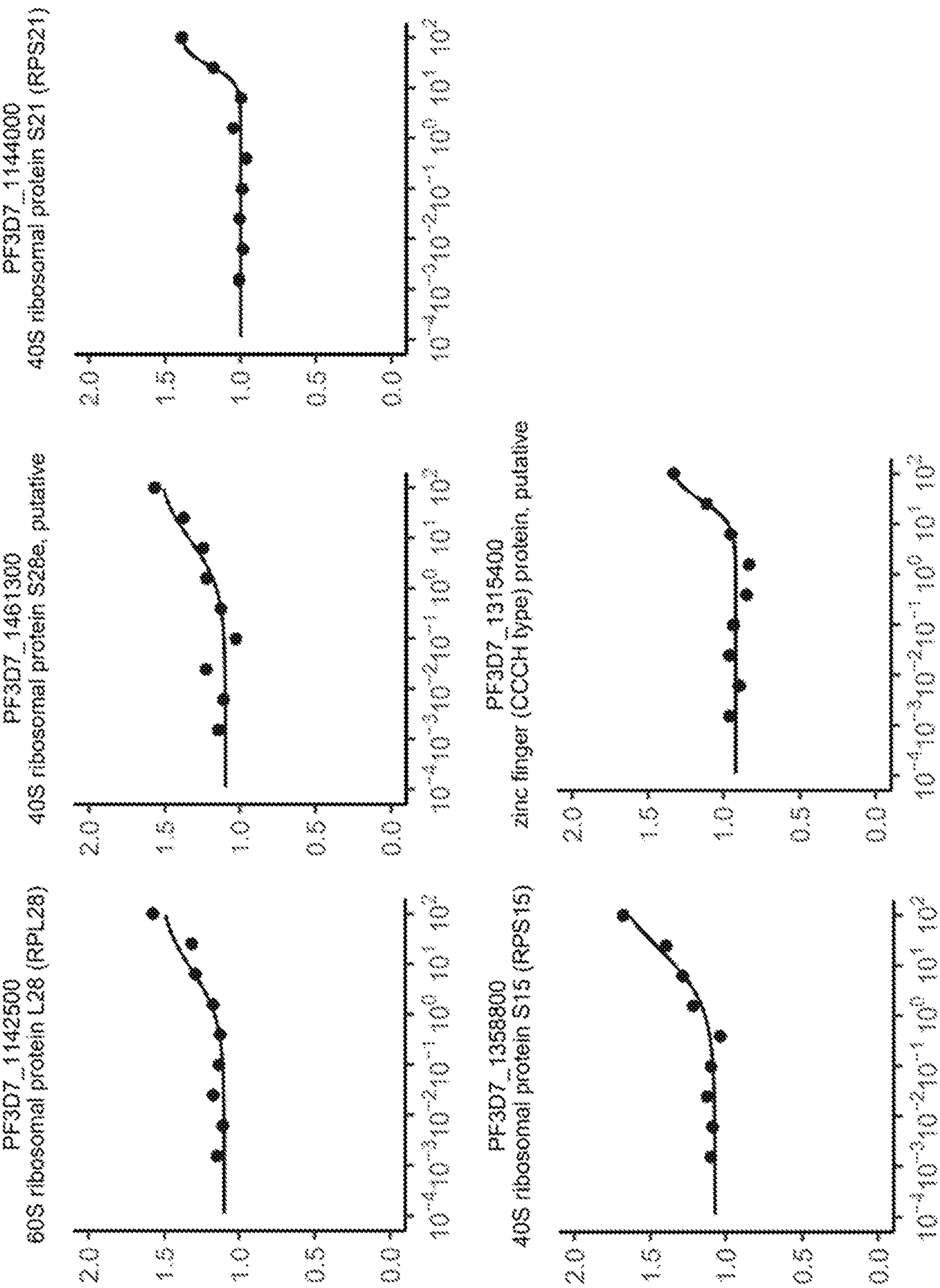
PF3D7_1142500
60S ribosomal protein L28 (RPL28)
PF3D7_1461300
40S ribosomal protein S28e, putative
PF3D7_1144000
40S ribosomal protein S21 (RPS21)
PF3D7_1358800
40S ribosomal protein S15 (RPS15)
PF3D7_1315400
zinc finger (CCCH type) protein, putative
2.0
1.5
1.0
0.5
0.0
$10^{-4}$ $10^{-3}$ $10^{-2}$ $10^{-1}$ $10^{0}$ $10^{1}$ $10^{2}$

Figure 4

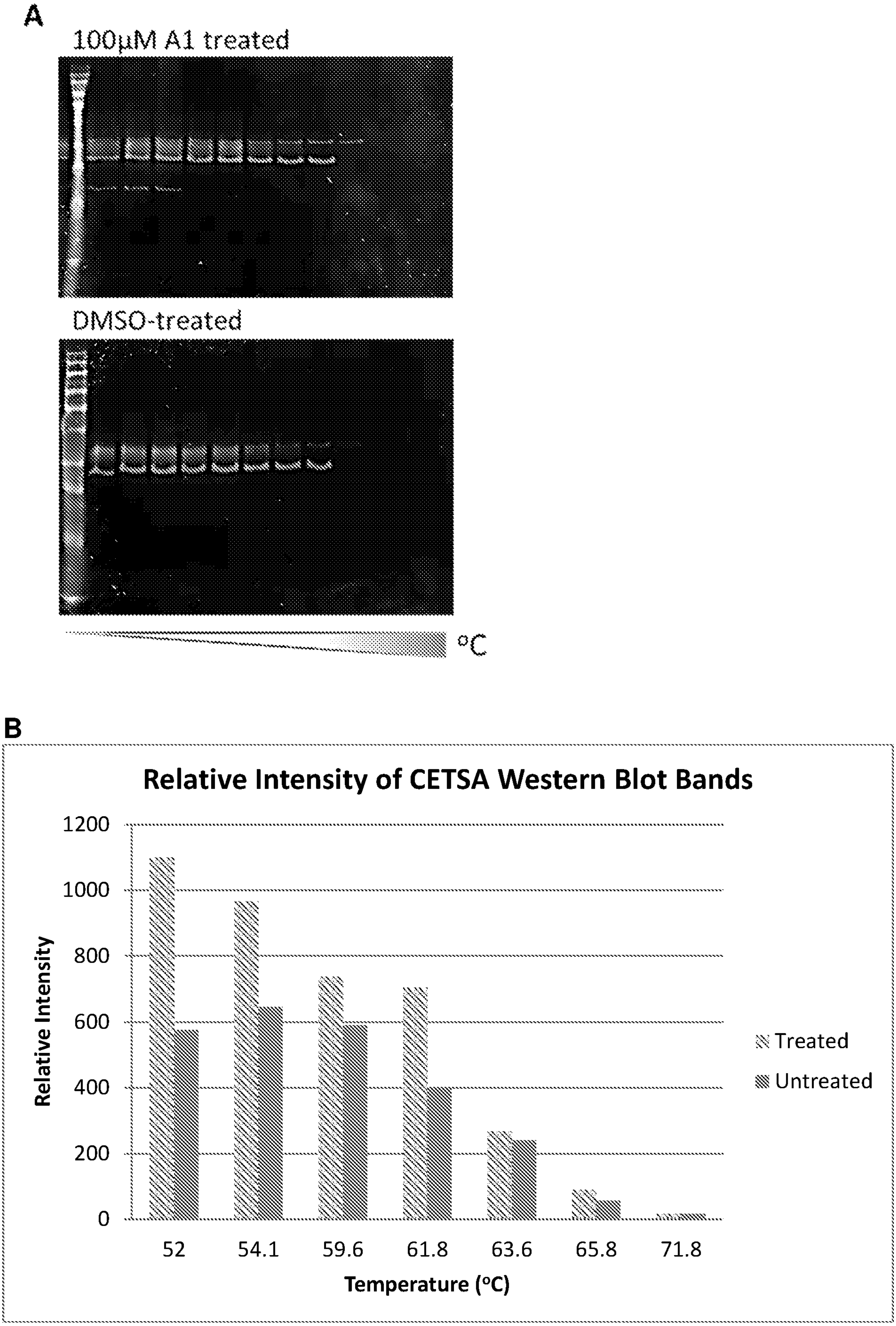
A
100µM A1 treated
DMSO-treated
°C
B
Relative Intensity of CETSA Western Blot Bands
Relative Intensity
1200
1000
800
600
400
200
0
52
54.1
59.6
61.8
63.6
65.8
71.8
Temperature (°C)
Treated
Untreated

Figure 5

C

| | Relative Band Intensity | |
|---|---|---|
| Temperature | Treated | Untreated |
| 51 | 876 | - |
| 52 | 1100 | 576 |
| 54.1 | 966 | 647 |
| 59.6 | 737 | 591 |
| 61.8 | 704 | 400 |
| 63.6 | 267 | 240 |
| 65.8 | 89.1 | 56.4 |
| 71.8 | 15.8 | 16.9 |

$(P < 0.05)$

Figure 5 (continued)

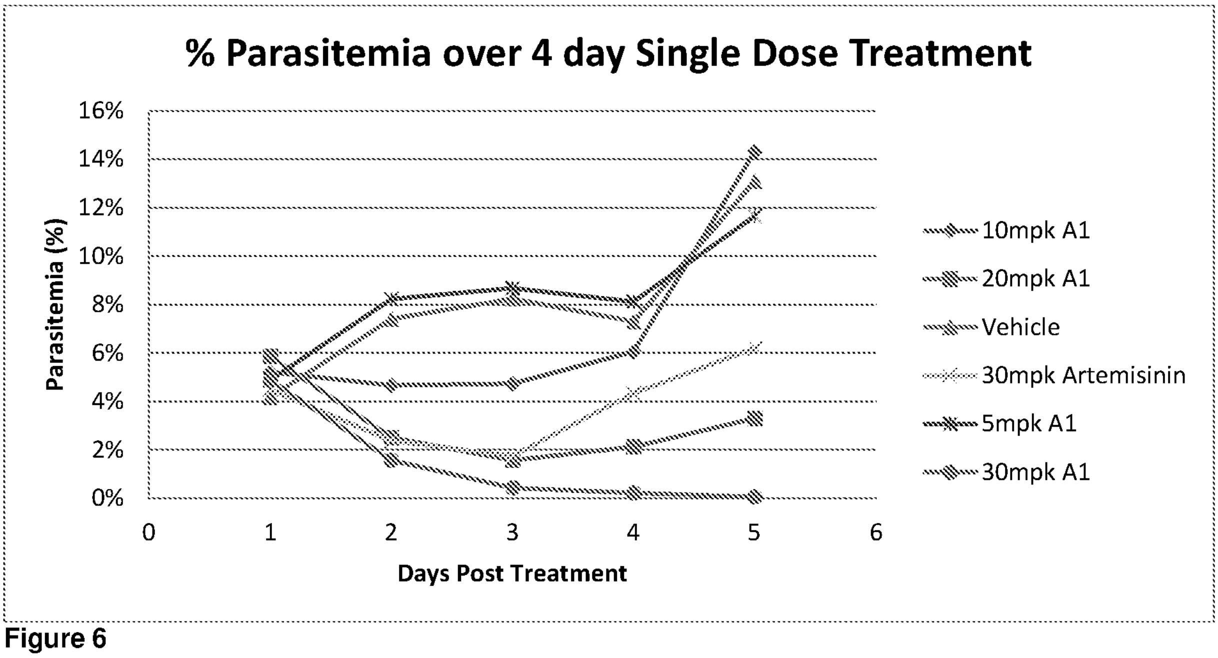


Figure 6

**A**

| Compound | Effect |
|---|---|
| 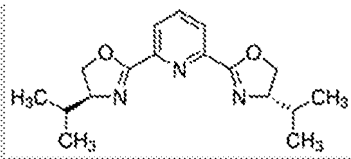 | No growth inhibition |
| 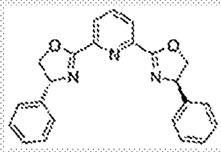 | 10% Inhibition at 10µM |
| 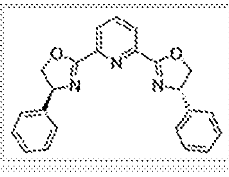 | 10-40% Inhibition at 10µM |
| 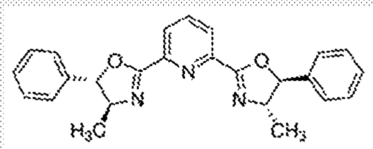 | 20% Inhibition at 10µM |
| 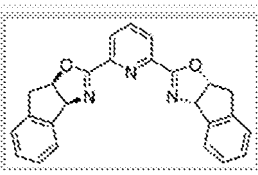 | No growth inhibition |

| Compound | Effect |
|---|---|
| 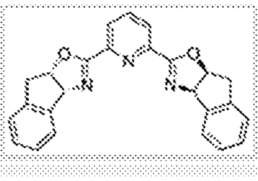 | No growth inhibition |
| 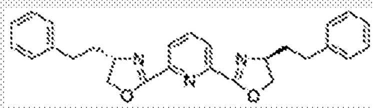 | 10-40% Inhibition at 10µM |
| 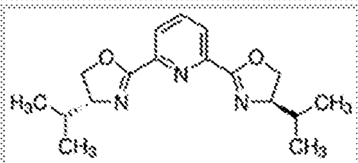 | No growth inhibition |
| 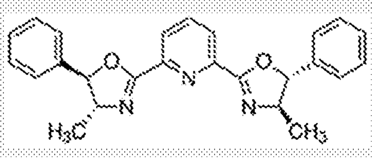 | No growth inhibition |

**Figure 7**

B

| Compound | $IC_{50}$(nM) |
|---|---|
| 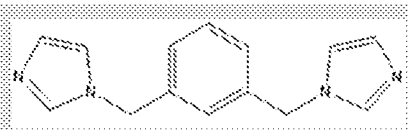 | No growth inhibition |
| 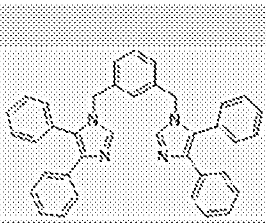 | 4686.667 |
| 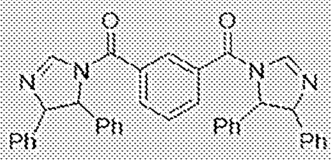 | 5042 |

| Compound | $IC_{50}$(nM) |
|---|---|
| 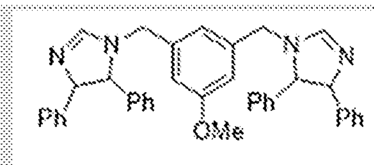 | 77.495 |
| 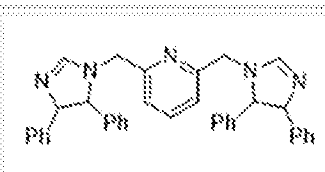 | 103.04 |
| 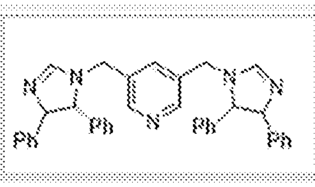 | 104.855 |

Figure 7 (continued)

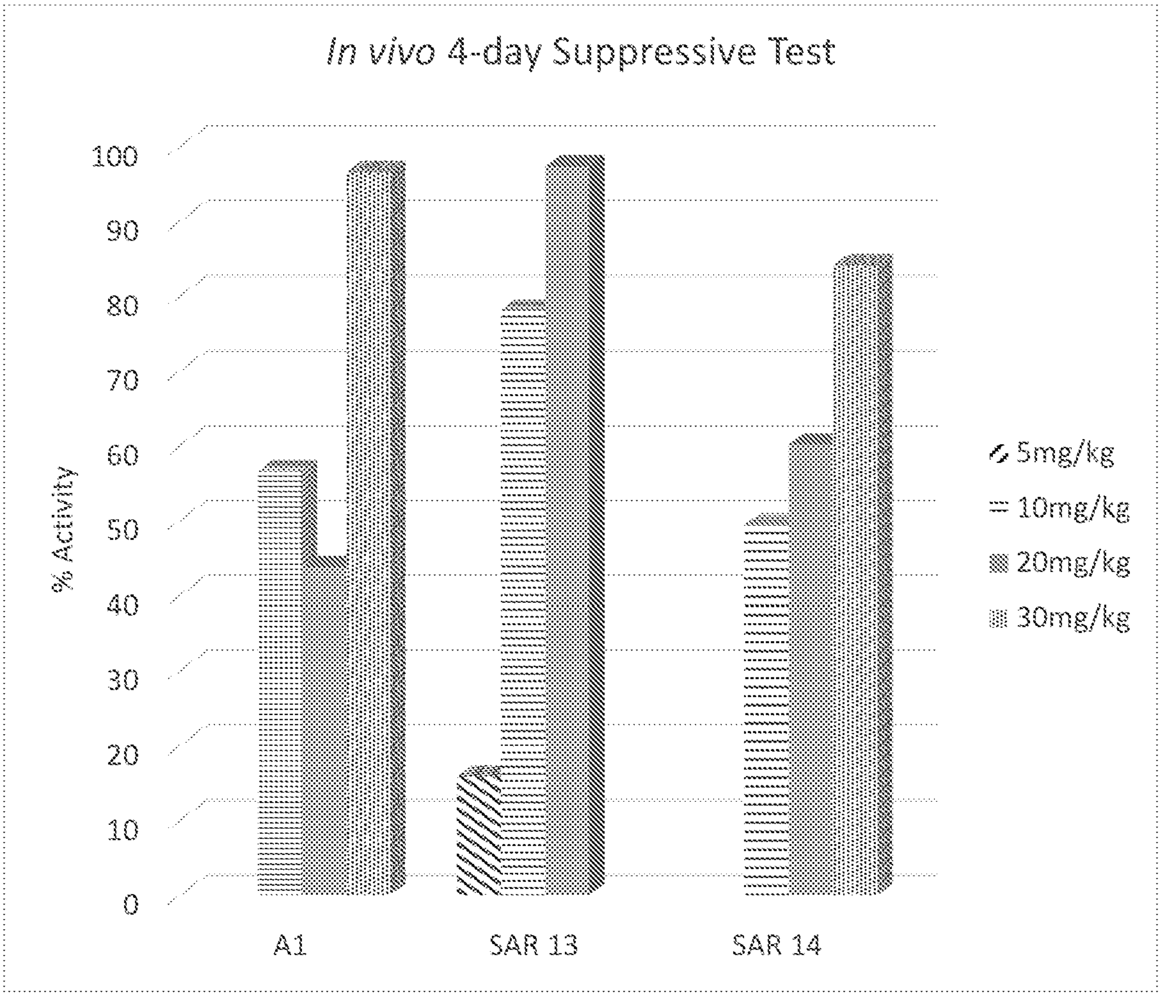
*In vivo* 4-day Suppressive Test
100
90
80
70
60
50
40
30
20
10
0
% Activity
A1
SAR 13
SAR 14
5mg/kg
10mg/kg
20mg/kg
30mg/kg

Figure 8

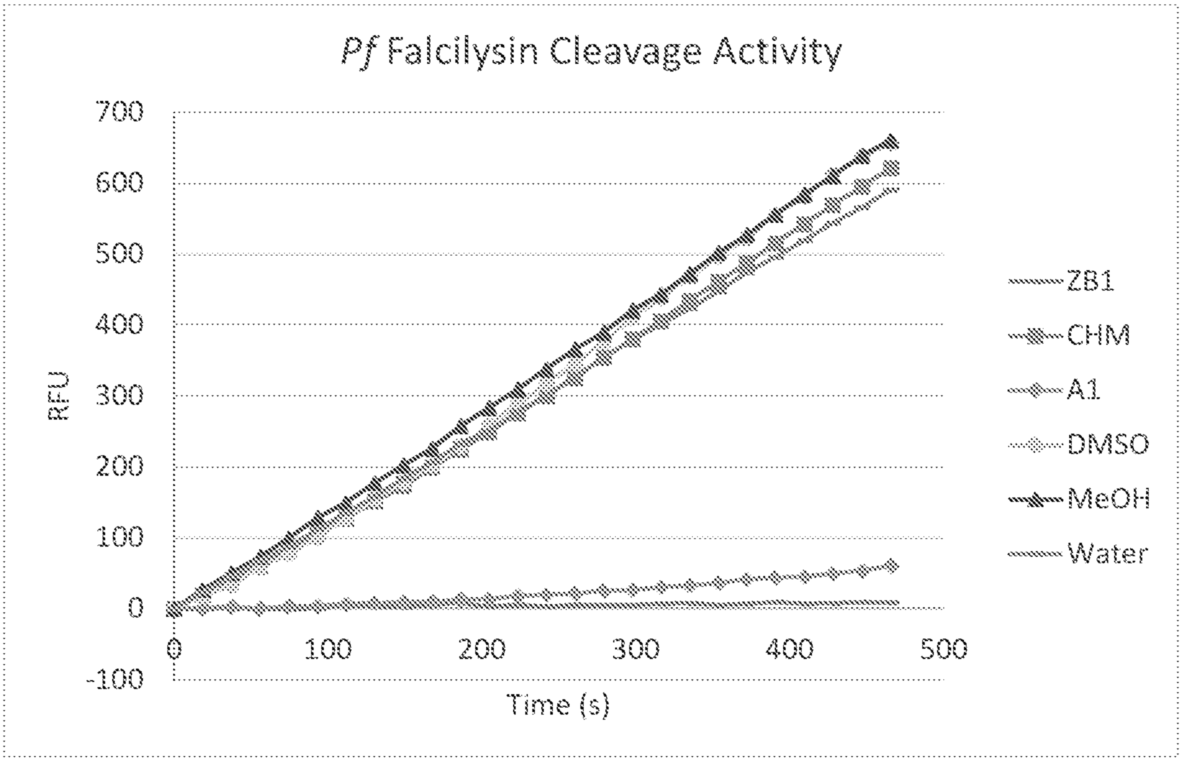
Pf Falcilysin Cleavage Activity
700
600
500
400
300
200
100
0
-100
RFU
0
100
200
300
400
500
Time (s)
ZB1
CHM
A1
DMSO
MeOH
Water

**Figure 9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017069601 A1
- WO 2009048586 A1
- WO 2003101954 A2
- WO 2014201161 A1
- WO 2009078992 A1


### Non-patent literature cited in the description


- **GOEBEL et al.** *J. Med. Chem.*, vol. 51 (2), 238-250
- **WORTH et al.** *J. Med. Chem.*, vol. 21 (4), 331-337
- **HE et al.** *Org. Lett.*, vol. 16 (12), 3244-3247
- **ÜSTÜN et al.** *Journal of Molecular Structure*, vol. 1123, 433-440
- **LU et al.** *Tetrahedron Letters*, vol. 53 (48), 6602-6605
- **SOBIA et al.** *Tetrahedron Asymmetry*, vol. 22 (16), 1632-1639
- **CHUANG et al.** *Chemistry-A European Journal*, vol. 15 (2), 405-417
- **JONES et al.** *Tetrahedron Letters*, vol. 42 (23), 3951-3954
- *J. Med. Chem.*, 2004, vol. 47 (10), 2393-404
- *Nat. Rev. Drug Discov.*, 2018, vol. 17 (8), 559-587
- **BERGE et al.** Pharmaceutical Salts. *Journal of Pharmaceutical Science*, 1977, vol. 66, 1-19
- **T. W. GREENE** ; **P. G. M. WUTS**. Protective Groups in Organic Chemistry. John Wiley and Sons, 1991
- **HE et al.** *Org. Lett. 2014*, 2014, vol. 16, 3244-3247
- **HE et al.** *Org. Lett.*, 2014, vol. 16, 3244-3247
- **MAIER** ; **RUG**. *Methods Mol Bio*, 2013, vol. 923, 3-15
- **DZIEKAN et al.** *Sci Transl Med 2019*, 2019, vol. 11 (473), eaau3174